# EUROPEAN PATENT APPLICATION

(11) **EP 3 023 103 A1**
(43) Date of publication of application: **25.05.2016**
(21) Application number: 14193862.1
(22) Date of filing: 19.11.2014
(51) Int. Cl.: A61K 36/78, A61K 36/882, A61K 36/9066, A61K 36/69, A61P 3/04

(54) **Pharmaceutical composition for anti-obesity comprising complex extracts including Saururi chinensis Baill. extract, Curcumae longae rhizoma extract and Polygalae radix extract**

(71) Applicant: Korea Institute of Oriental Medicine, Daejeon 305-811 (KR)
(72) Inventor: Ma, Jin Yeul, 305-735 Daejeon (KR); Kwak, Dong Hoon, 305-800 Daejeon (KR); Lee, Ji Hye, 305-810 Daejeon (KR); Kim, Dong Gun, 305-810 Daejeon (KR); Kim, Tae Soo, 305-761 Daejeon (KR)
(74) Representative: Regimbeau

(57) **Abstract**

Disclosed herein are a pharmaceutical composition for the prevention or treatment of obesity comprising an extract of a mixture comprising Saururi chinensis Baill., Curcumae Longae Rhizoma, and Polygalae Radix, or an extract of a mixture comprising Saururi chinensis Baill., Curcumae Longae Rhiwma, Polygalae Radix, and Acori Gramineri Rhizoma; a food composition comprising the extract of the mixture; and a method of manufacturing the extracts of the mixture. The extracts of the present invention can inhibit fat generation without adverse effects such as cellular toxicity and thus can be widely used for the prevention, improvement, or treatment of obesity or obesity-related diseases.

## Description

### BACKGROUND OF THE INVENTION

### 1. Field ofthe Invention

The present invention relates generally to a composition for preventing or treating obesity comprising an extract of a mixture comprising Saururi chinensis Baill., Curcumae Longae Rhizoma, and Polygalae Radix, and more particularly, to a pharmaceutical composition and a food composition for preventing or treating obesity comprising an extract of a mixture comprising Saururi chinensis Baill., Curcumae Longae Rhizoma, and Polygalae Radix, or an extract of a mixture comprising Saururi chinensis Baill., Curcumae Longae Rhizoma, Polygalae Radix and Acori Gramineri Rhizoma, and a method for manufacturing the extracts.

### 2. Description of the Related Art

As well known in the art, obesity generally refers to a medical condition of excess fat accumulation in the body. Obesity is a phenomenon where the energy surplus resulting from an imbalance between energy input from food intake and energy consumption by physical acitivities accumulates in the body as body fat. A long-term abnormal accumulation of body fat resulting from an energy surplus can cause various metabolic diseases and adult diseases such as diabetes, hyperlipidemia, heart diseases, strokes, atherosclerosis, and fatty liver, and has become a serious global issue

Obesity can occur due to various reasons, including genetic factors, environmental factors such as westernized dietary patterns, psychological factors due to stresses, disorders in energy metabolism, etc. The types of obesity-causing factors maybe divided into simple obesity due to overeating and lack of exercise, and symptomatic obesity such as endocrine obesity, hypothalamic obesity, genetic obesity, and metabolic obesity.

The degree of obesity can be evaluated by measuring body weight or the thickness of skin folds. In general, a person with an obesity index of 25 or higher is defined as being obese. Obesity index (body mass index; BMI) is a value obtained by dividing a person's body weight (in kg) over the square of his/her height (m²). For westerners, people with a BMI of 30 or higher are considered obese, and in South Korea, people with a BMI of 25 or higher are considered obese considering racial differences.

Multilateral studies have been performed globally in order to develop therapeutic agents for treating obesity. The drugs for treating obesity, depending on their working mechanisms, may be largely divided into those for inhibiting fat absorption, promoting fat decomposition and heat generation, controlling appetite and satiety, inhibiting protein metabolism, and controlling emotions relating to food intake. Examples of representative therapeutic agents for obesity include Xenical™, which, being prepared using orlistat as a raw ingredient, inhibits fat absorption, and Reductil™, which, being prepared using sibutramine as a main raw ingredient, inhibits appetite by stimulating the sympathetic nervous system. However, Xenical™ is known to have side effects such as steartorrhea, abdominal pain, vomiting, pruritus, and liver damage, whereas Reductil™ is known to have side effects such as headaches, anorexia, insomnia, and constipation, and also serious cardiovascular diseases, and thus their administration guidelines have been recently strengthened.

In addition to drug therapies, there are other therapies for preventing and treating obesity such as diet therapy to restrict food intake, exercise therapy to increase energy consumption, psychotherapy, behavior therapy, and surgical therapy.

Preferably, a combined therapy of exercising to promote energy consumption while administrating a therapeutic drug for treating obesity with few side effects is suggested as the safest and most effective method for the treatment of obesity. However, the saftety issue on the therapeutic drugs for treating obesity has not been resolved as described above in the serious side effects of Xenical™ and Reductil™. Accordingly, there is a need to develop a substance capable with advanced safety as well as anti-obesity efficacies in the human body.

As such, the present inventors, while endeavoring to find a substance having an excellent therapeutic effect on obesity without any side effects in the human body, discovered that an extract of a mixture comprising Saururi chinensis Baill., Curcumae Longae Rhizoma, and Polygalae Radix, exhibits effects of preventing and treating obesity within the range of not causing cell toxicity, thereby completing the present invention.

### SUMMARY OF THE INVENTION

Accordingly, the present invention has been made keeping in mind the above problems occurring in the prior art, and an objective of the present invention is to provide a method for preventing and treating obesity comprising administering an effective amount of an extract of a mixture comprising Saururi chinensis Baill., Curcumae Longae Rhizoma, and Polygalae Radix; or a fraction thereof to a subject in need thereof

Another objective of the present invention is to provide a method for the prevention and treatment of obesity comprising administering an effective amount of an extract of a mixture comprising Saururi chinensis Baill., Curcumae Longae Rhizoma, Polygalae Radix, and Acori Gramineri Rhizoma; or a fraction thereof to a subject in need thereof.

Still another objective of the present invention is to provide a method of manufacturing an extract from a mixture comprising Saururi chinensis Baill., Curcumae Longae Rhizoma, and Polygalae Radix by extracting with water, a C₁ ∼ C₄ alcohol, or a mixed solvent thereof

Still another objective of the present invention is to provide a method of manufacturing an extract from a mixture comprising Saururi chinensis Baill., Curcumae Longae Rhizoma, Polygalae Radix, and Acori Gramineri Rhizoma by extracting with water, a C₁ ∼ C₄ alcohol, or a mixed solvent thereof

Still another objective of the present invention is to provide a composition comprising an extract of a mixture comprising Saururi chinensis Baill., Curcumae Longae Rhizoma, and Polygalae Radix; or a fraction thereof

Still another objective of the present invention is to provide a composition comprising an extract of a mixture comprising Saururi chinensis Baill., Curcumae Longae Rhizoma, Polygalae Radix, and Acori Gramineri Rhizoma; or a fraction thereof

In an aspect of the present invention, in order to accomplish the above objectives, the present invention provides a pharmaceutical composition for the prevention and treatment of obesity comprising an extract of a mixture comprising Saururi chinensis Baill., Curcumae Longae Rhizoma, and Polygalae Radix; an extract of a mixture comprising Saururi chinensis Baill., Curcumae Longae Rhizoma, Polygalae Radix, and Acori Gramineri Rhizoma; or a fraction thereof.

### BREIF DESCRIPTION OF THE DRAWINGS

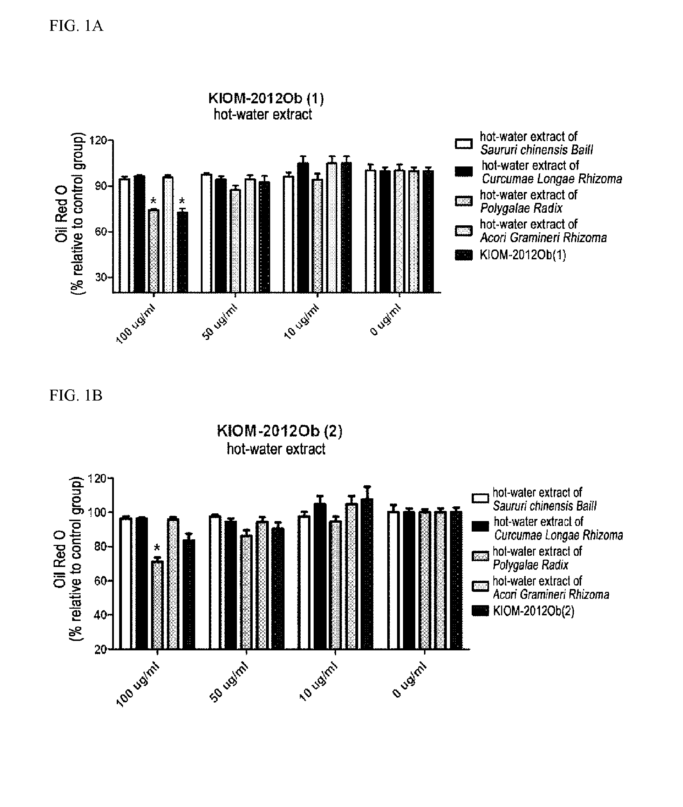

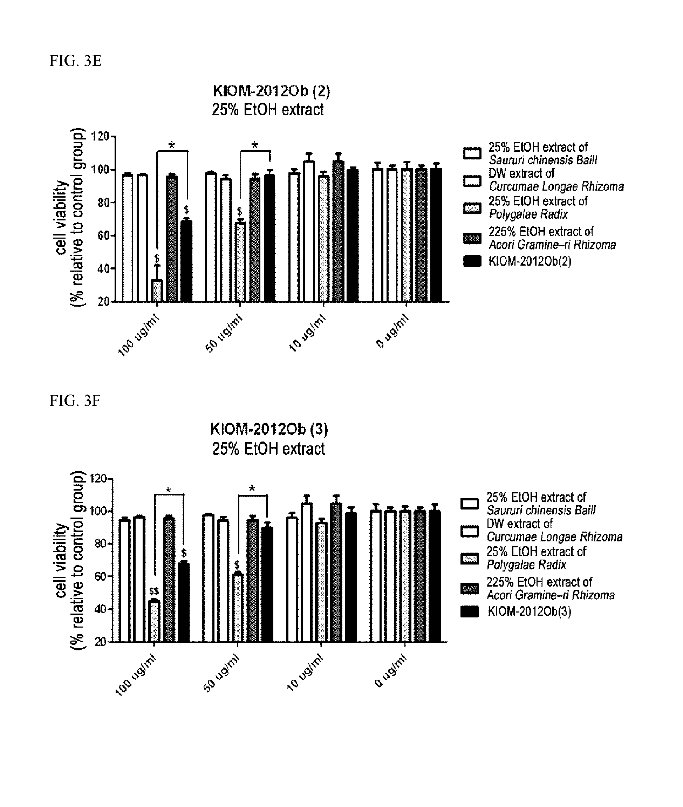

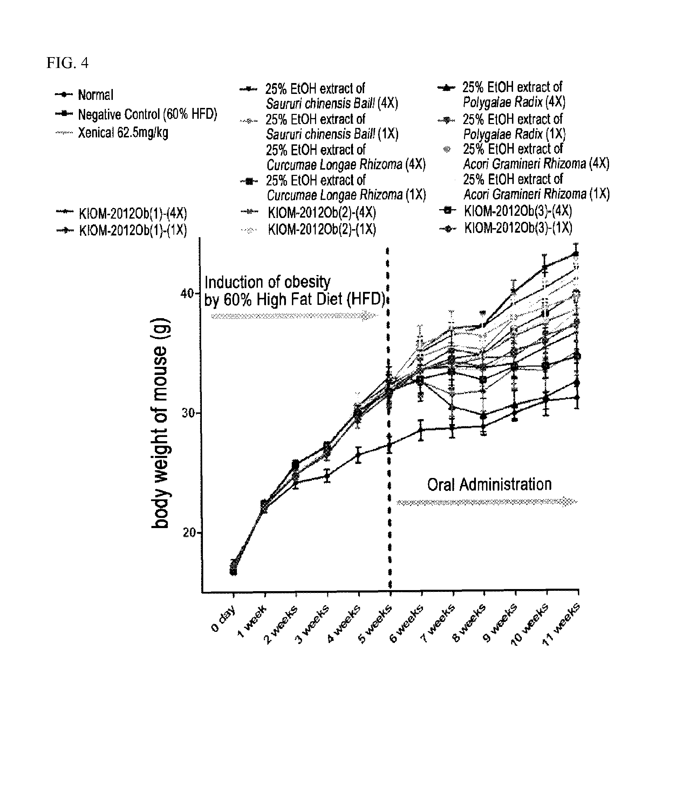

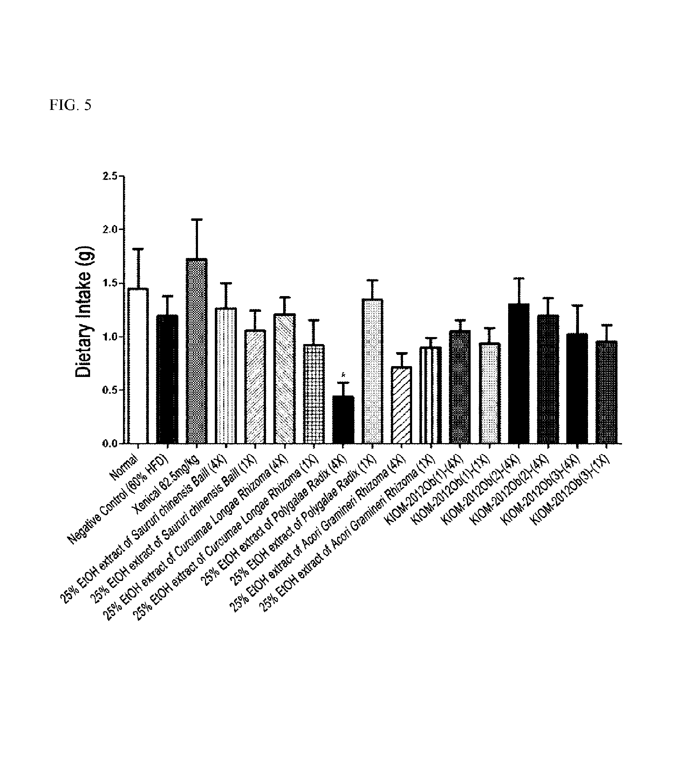

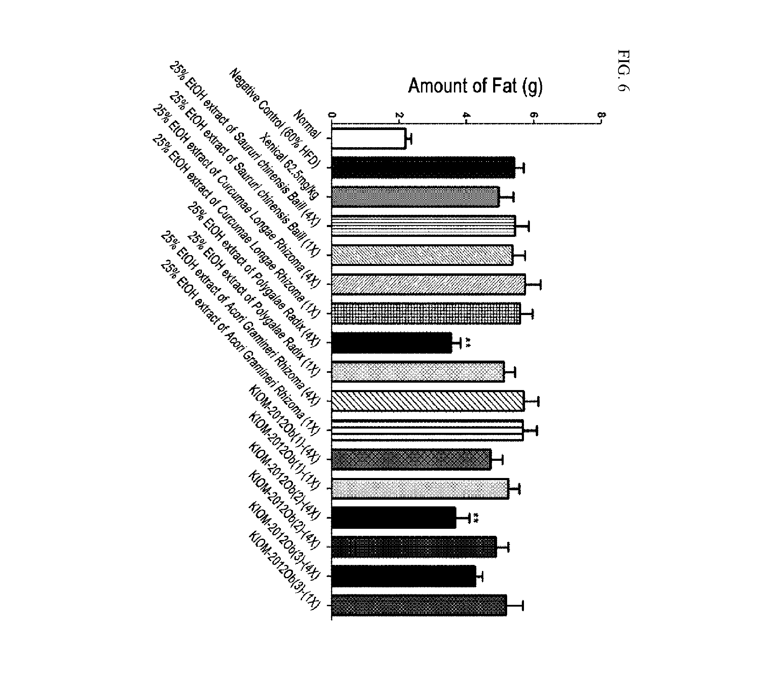

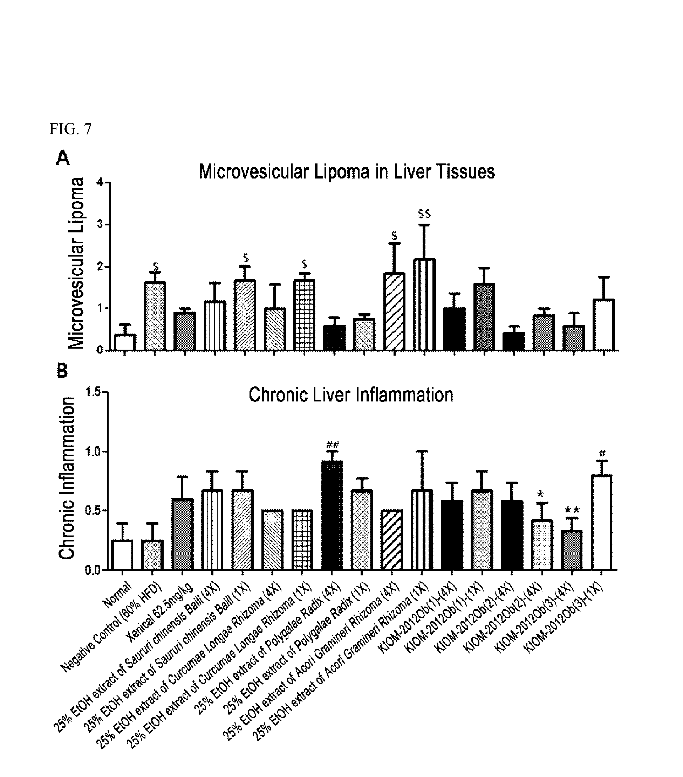

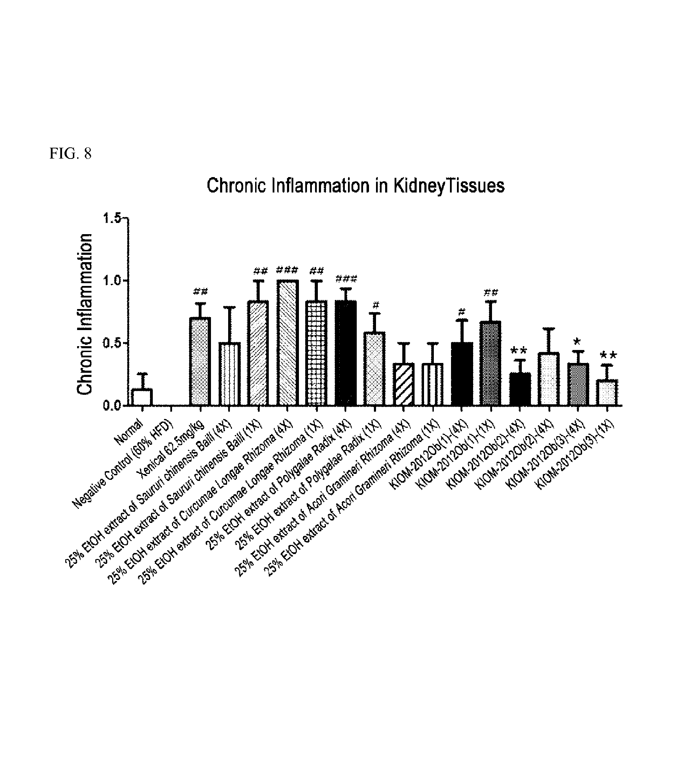

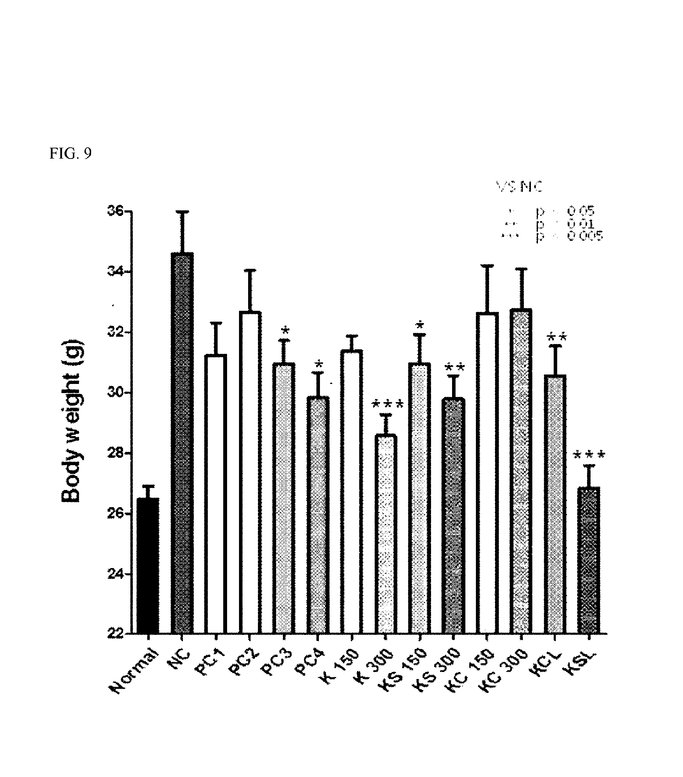

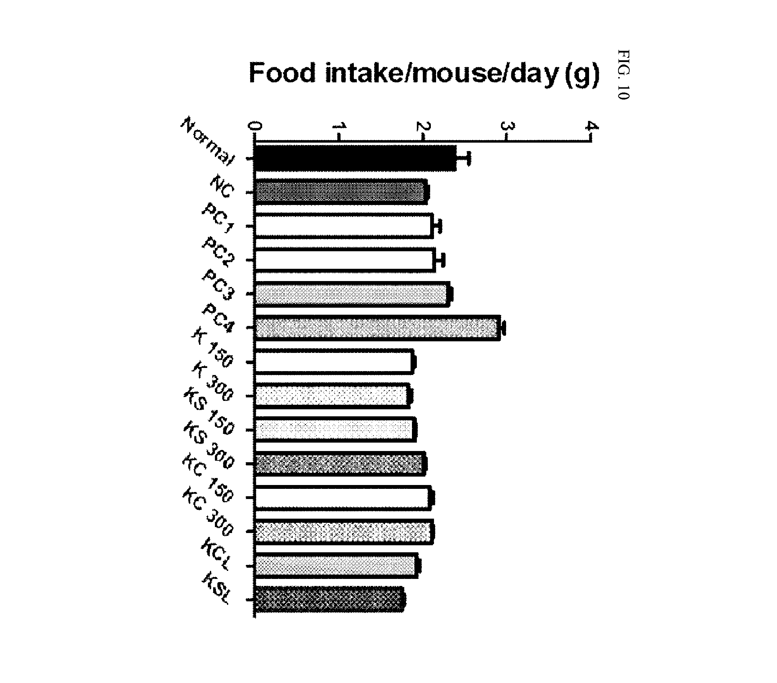

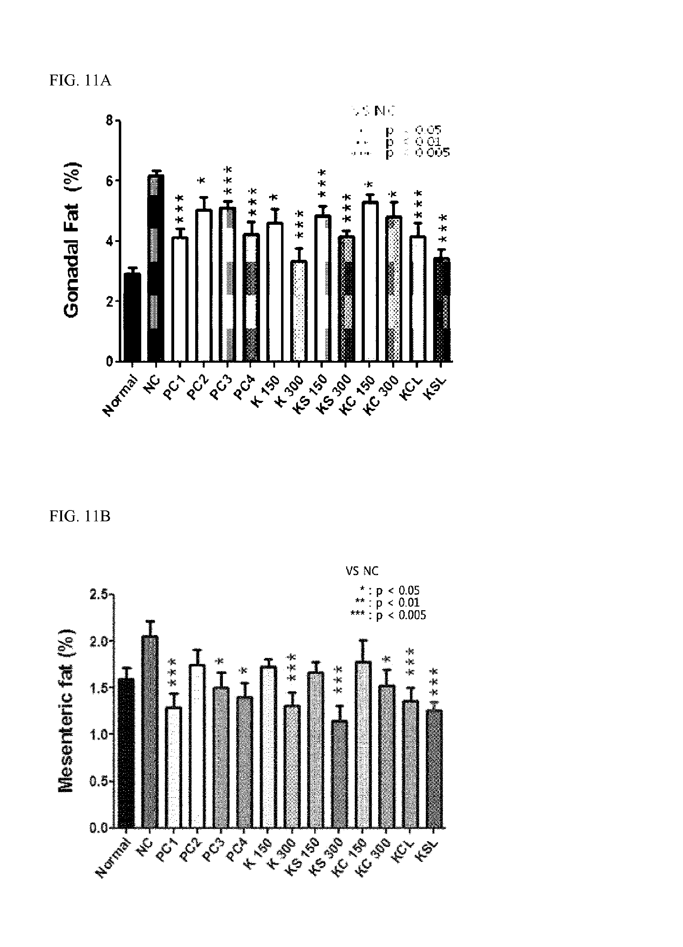

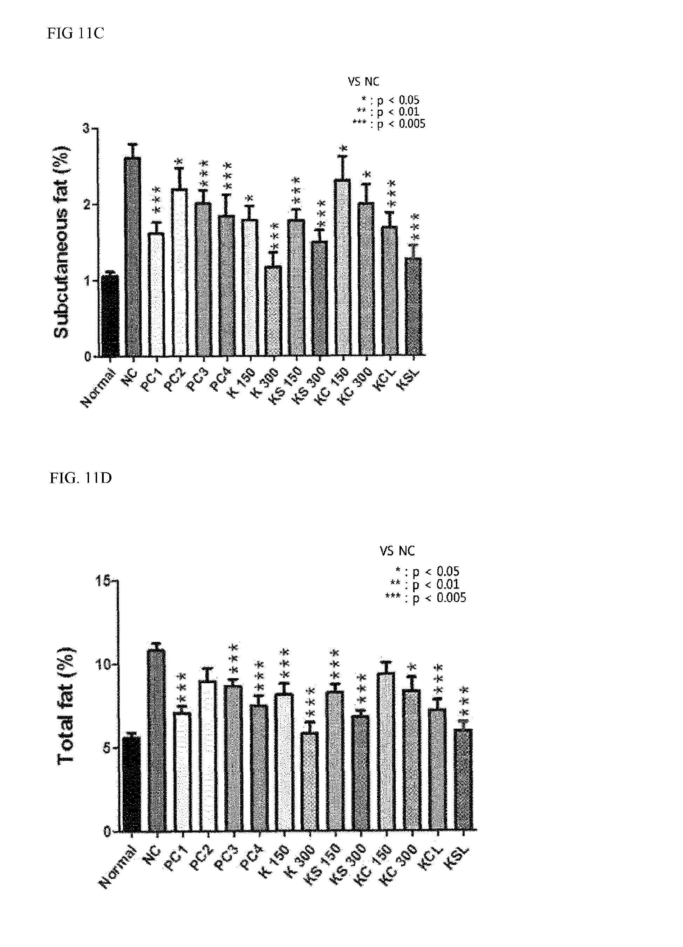

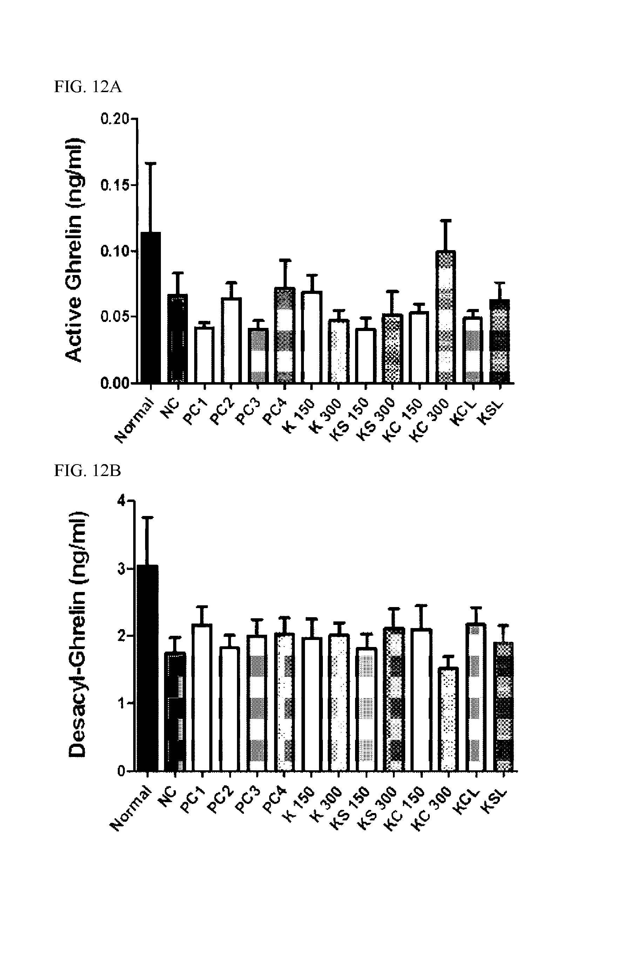

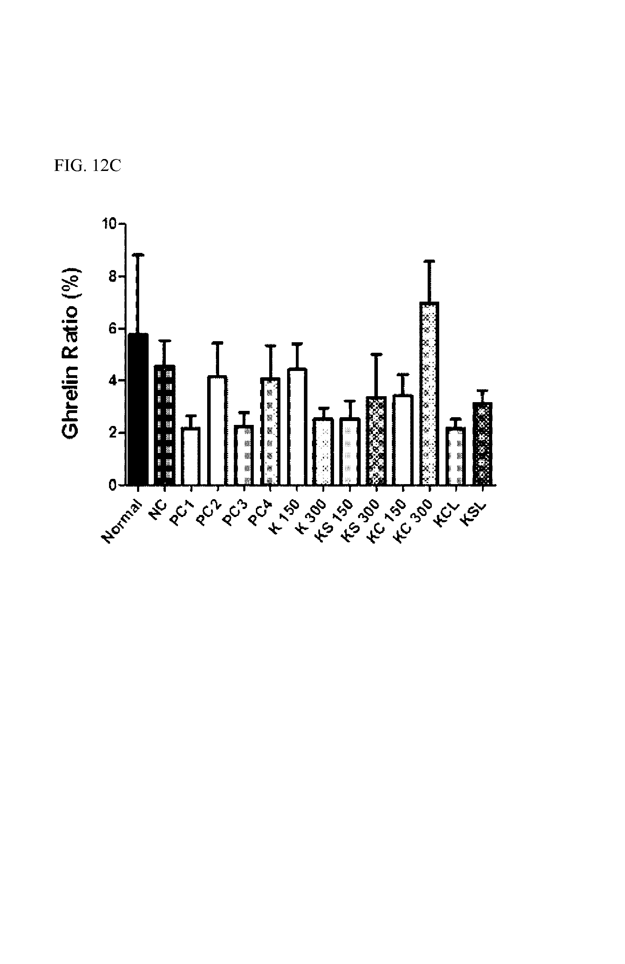

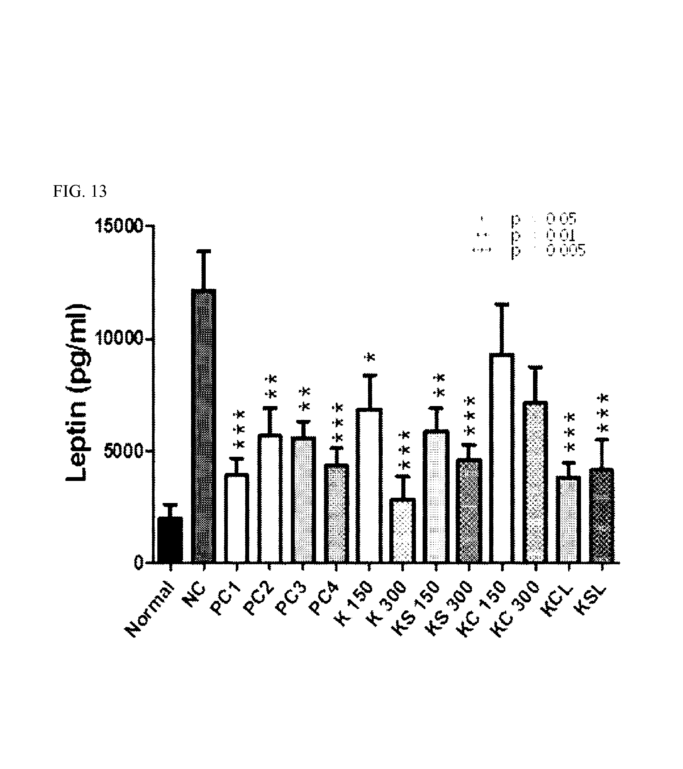

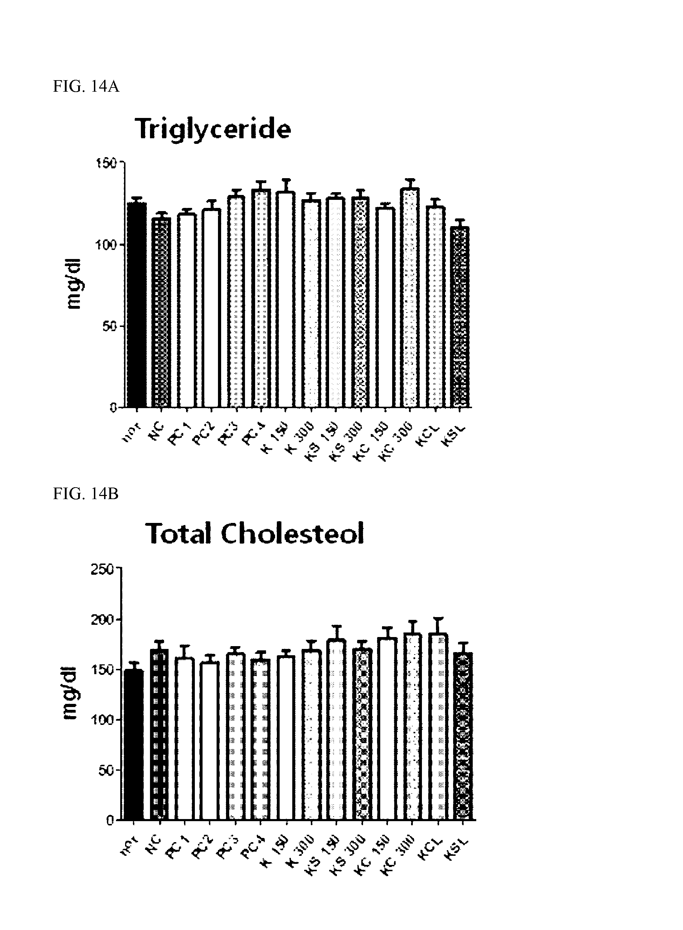

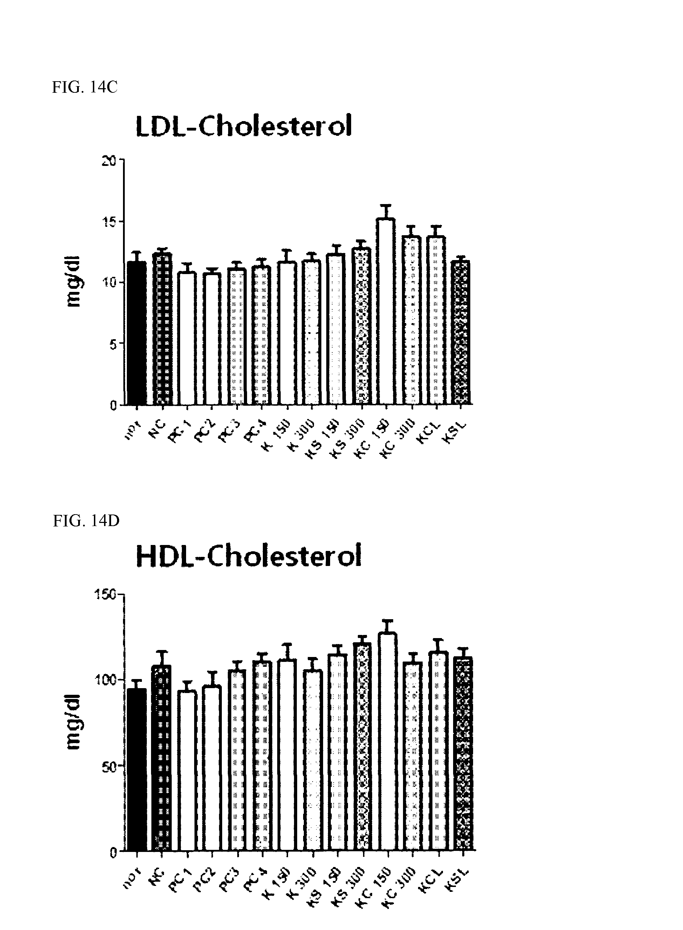

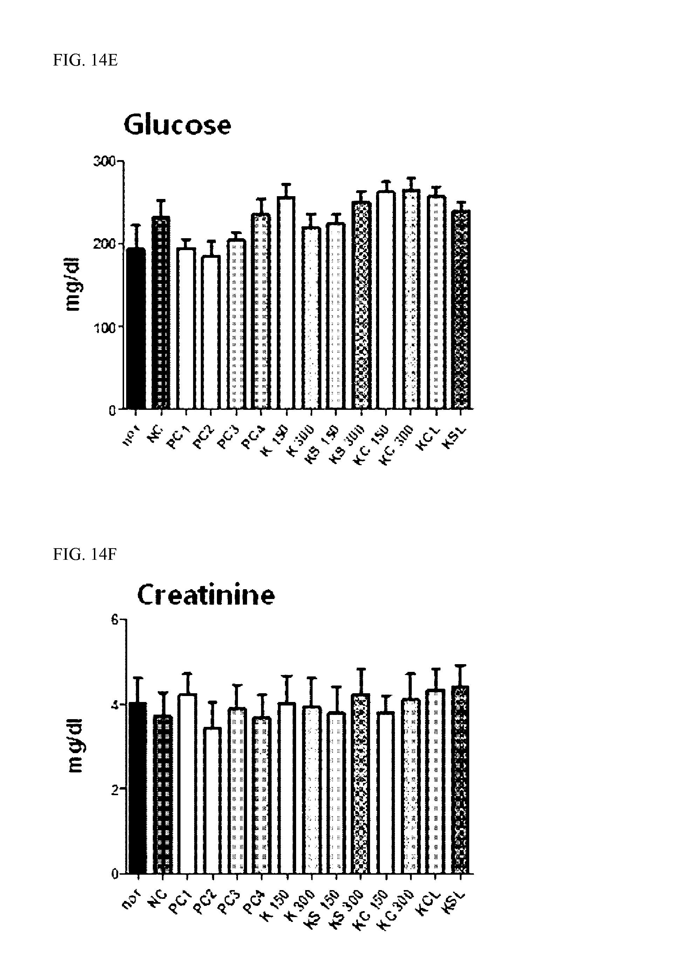

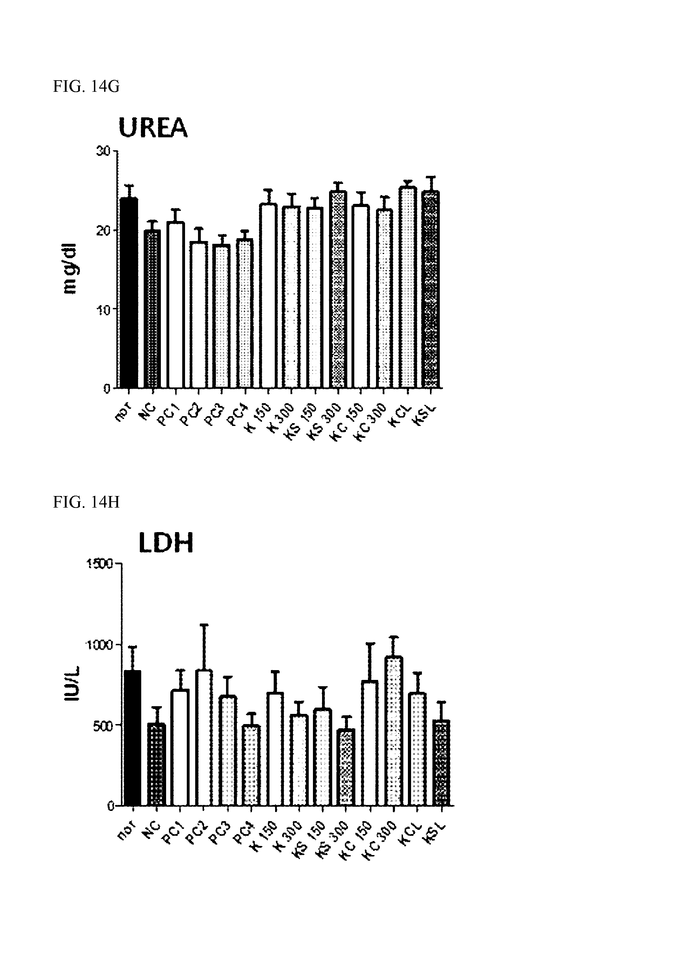

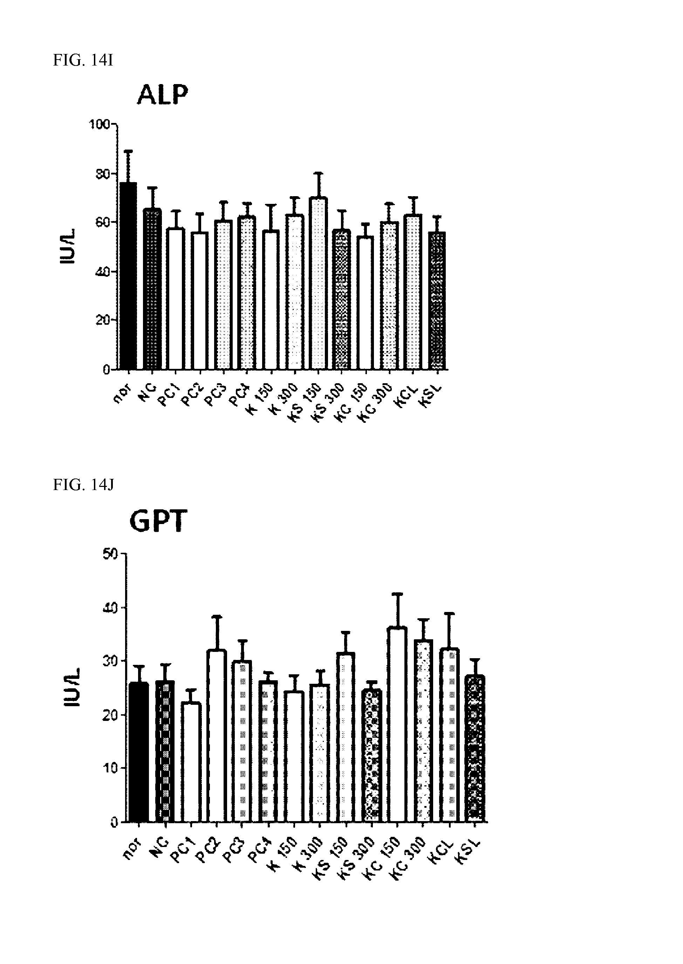

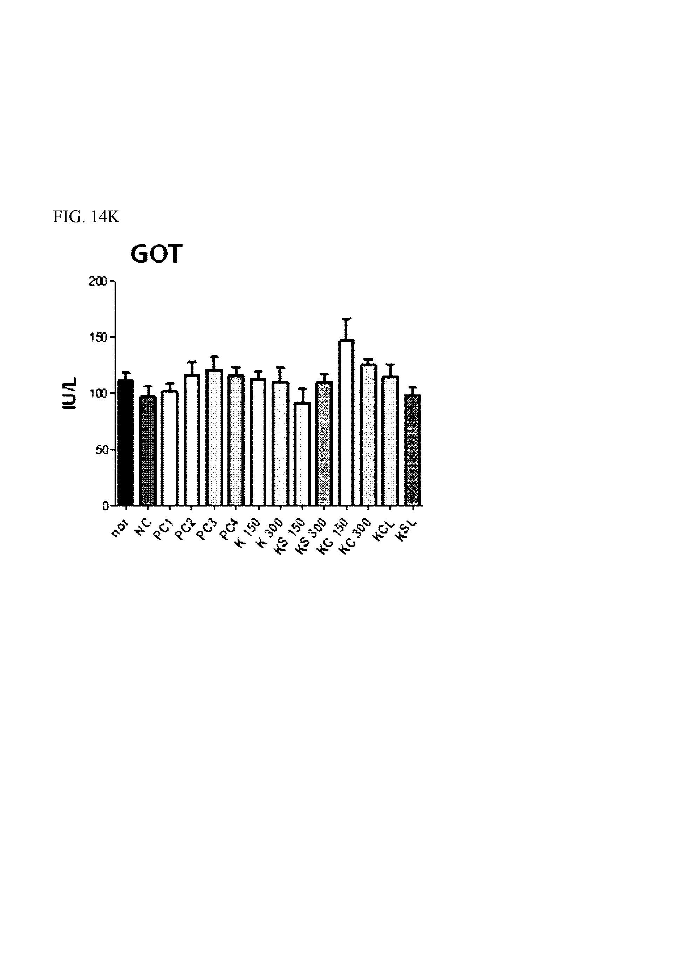

FIG. 1A is a graph illustrating the inhibitory effects of KIOM-2012Ob(1) hot-water extracts against the accumulation of triglycerides within the adipocytes differentiated from 3T3-L1 via Oil Red O Staining. The Oil Red O Staining was performed 8 days after treating the cells with the KIOM-2012Ob(1) and each of the hot-water extracts of the herbal components constituted in the KIOM-2012Ob(1) while inducing differentiation from 3T3-L1. In the graph (FIG 1A), the treatment concentration was shown as the concentration of KIOM-2012Ob(1), and Saururi chinensis Baill., Curcumae Longae Rhizoma, Polygalae Radix, and Acori Gramineri Rhizoma constituted in KIOM-2012Ob(1) were treated in the same manner as in the contents of the composition. '*' denotes the significance (P<0.05) of the decrease in the accumulation of triglycerides relative to that of the control group (0 µg/mL).
FIG. 1B is a graph illustrating the inhibitory effects of KIOM-2012Ob(2) hot-water extracts against the accumulation of triglycerides within the adipocytes differentiated from 3T3-L1 via Oil Red O Staining. The Oil Red O Staining was performed 8 days after treating the cells with the KIOM-2012Ob(2) and each of the hot-water extracts of the herbal components constituted in the KIOM-2012Ob(2) while inducing differentiation from 3T3-L1. In the graph (FIG 1B), the treatment concentration was shown as the concentration of KIOM-2012Ob(2), and Saururi chinensis Baill., Curcumae Longae Rhizoma, Polygalae Radix, and Acori Gramineri Rhizoma constituted in KIOM-2012Ob(2) were treated in the same manner as in the contents of the composition. '*' denotes the significance (P<0.05) of the decrease in the accumulation of triglycerides relative to that of the control group (0 µg/mL).

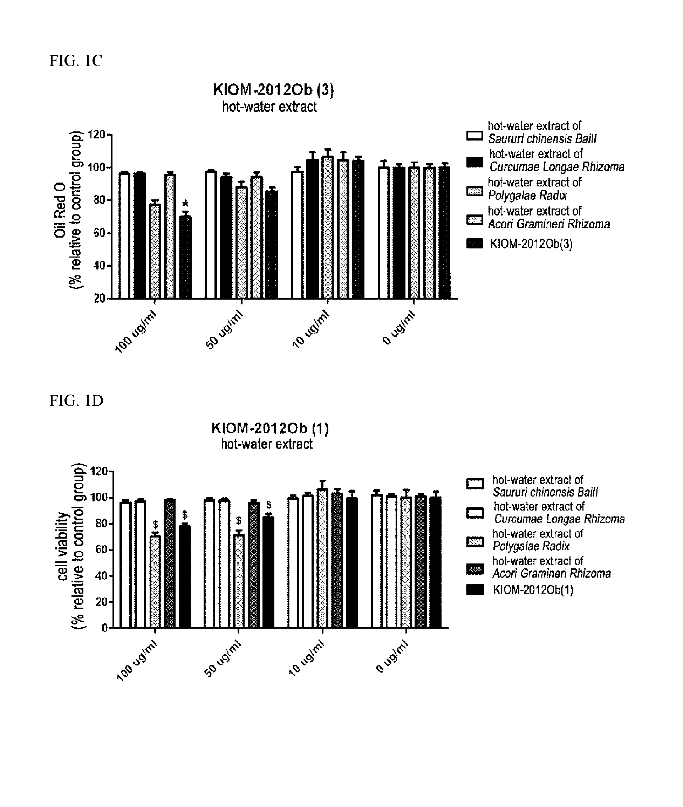

FIG. 1C is a graph illustrating the inhibitory effects of KIOM-2012Ob(3) hot-water extracts against the accumulation of triglycerides within the adipocytes differentiated from 3T3-L1 via Oil Red O Staining. The Oil Red O Staining was performed 8 days after treating the cells with the KIOM-2012Ob(3) and each of the hot-water extracts of the herbal components constituted in the KIOM-2012Ob(3) while inducing differentiation from 3T3-L1. In the graph, the treatment concentration was shown as the concentration of KIOM-2012Ob(3), and Saururi chinensis Baill., Curcumae Longae Rhizoma, Polygalae Radix, and Acori Gramineri Rhizoma constituted in KIOM-2012Ob(3) were treated in the same manner as in the contents of the composition. '*' denotes the significance (P<0.05) of the decrease in accumulation of triglycerides relative to that of the control group (0 µg/mL).
FIG. 1D is a graph illustrating the effect of KIOM-2012Ob(1) hot-water extracts on the viability of the cells differentiated from 3T3-L1. The viability of the cells differentiated from 3T3-L1 was shown after measurement via WST method. In the graph, the treatment concentration was shown as the concentration of KIOM-2012Ob(1), and Saururi chinensis Baill., Curcumae Longae Rhizoma, Polygalae Radix, and Acori Gramineri Rhizoma constituted in KIOM-2012Ob(1) were treated in the same manner as in the contents of the composition. '$' denotes the significance (P<0.05) of the decrease of the cell viability relative to that of the control group (0 µg/mL).

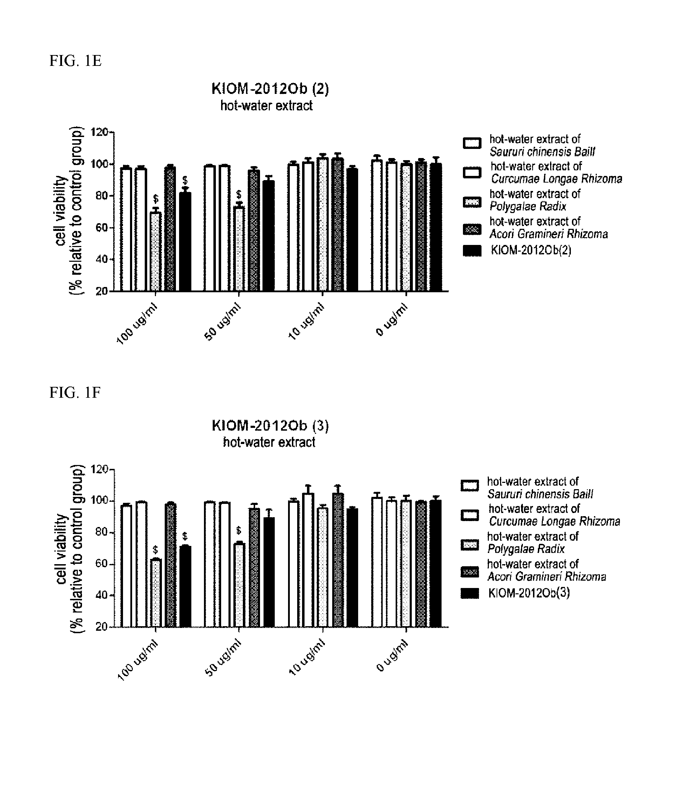

FIG. 1E is a graph illustrating the effect of KIOM-2012Ob(2) hot-water extract on the viability of the cells differentiated from 3T3-L1. The viability of the cells differentiated from 3T3-L1 was shown after measurement via WST method. In the graph, the treatment concentration was shown as the concentration of KIOM-2012Ob(2), and Saururi chinensis Baill., Curcumae Longae Rhizoma, Polygalae Radix, and Acori Gramineri Rhizoma constituted in KIOM-2012Ob(2) were treated in the same manner as in the contents of the composition. '$' denotes the significance (P<0.05) of the decrease of the cell viability relative to that of the control group (0 µg/mL).
FIG. 1F is a graph illustrating the effect of KIOM-2012Ob(3) hot-water extract on the viability of the cells differentiated from 3T3-L1. The viability of the cells differentiated from 3T3-L1 was shown after measurement via WST method. In the graph, the treatment concentration was shown as the concentration of KIOM-2012Ob(3), and Saururi chinensis Baill., Curcumae Longae Rhizoma, Polygalae Radix, and Acori Gramineri Rhizoma constituted in KIOM-2012Ob(3) were treated in the same manner as in the contents of the composition. '$' denotes the significance (P<0.05) of the decrease of the cell viability relative to that of the control group (0 µg/mL).

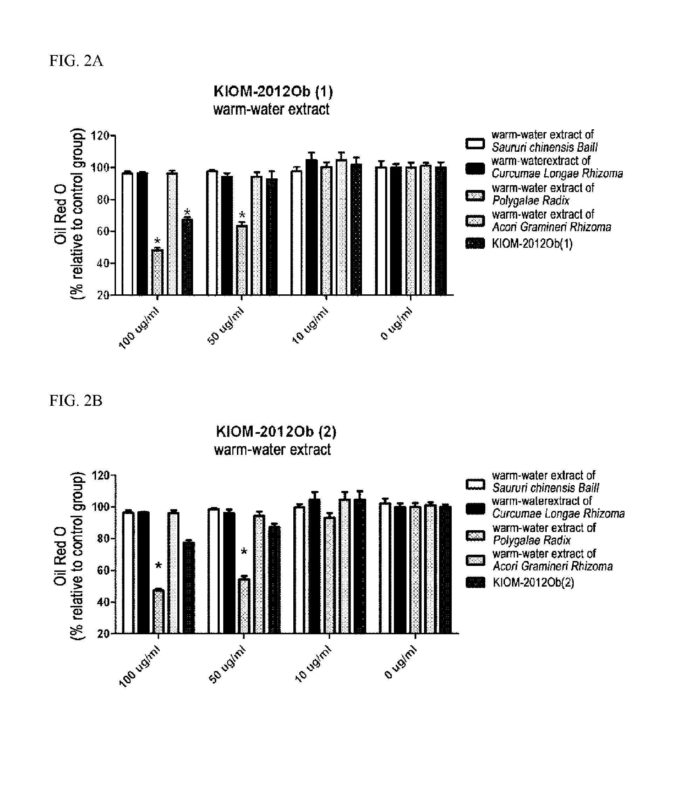

FIG. 2A is a graph illustrating the inhibitory effects of KIOM-2012Ob(1) warm-extracts against the accumulation of triglycerides within the adipocytes differentiated from 3T3-L1 via Oil Red O Staining. The Oil Red O Staining was performed 8 days after treating the cells with the KIOM-2012Ob(1) and each of the warm-water extracts of the herbal components constituted in the KIOM-2012Ob(1) while inducing differentiation from 3T3-L1. In the graph, the treatment concentration was shown as the concentration of KIOM-2012Ob(1), and Saururi chinensis Baill., Curcumae Longae Rhizoma, Polygalae Radix, and Acori Gramineri Rhizoma constituted in KIOM-2012Ob(1) were treated in the same manner as in the contents of the composition. '*' denotes the significance (P<0.05) of the decrease in accumulation of triglycerides relative to that of the control group (0 µg/mL).
FIG. 2B is a graph illustrating the inhibitory effects of KIOM-2012Ob(2) warm-water extracts against the accumulation of triglycerides within the adipocytes differentiated from 3T3-L1 via Oil Red O Staining. The Oil Red O Staining was performed 8 days after treating the cells with the KIOM-2012Ob(2) and each of the warm-water extracts of the herbal components constituted in the KIOM-2012Ob(2) while inducing differentiation from 3T3-L1. In the graph (FIG 2B), the treatment concentration was shown as the concentration of KIOM-2012Ob(2), and Saururi chinensis Baill., Curcumae Longae Rhizoma, Polygalae Radix, and Acori Gramineri Rhizoma constituted in KIOM-2012Ob(2) were treated in the same manner as in the contents of the composition. '*' denotes the significance (P<0.05) of the decrease in accumulation of triglycerides relative to that of the control group (0 µg/mL).

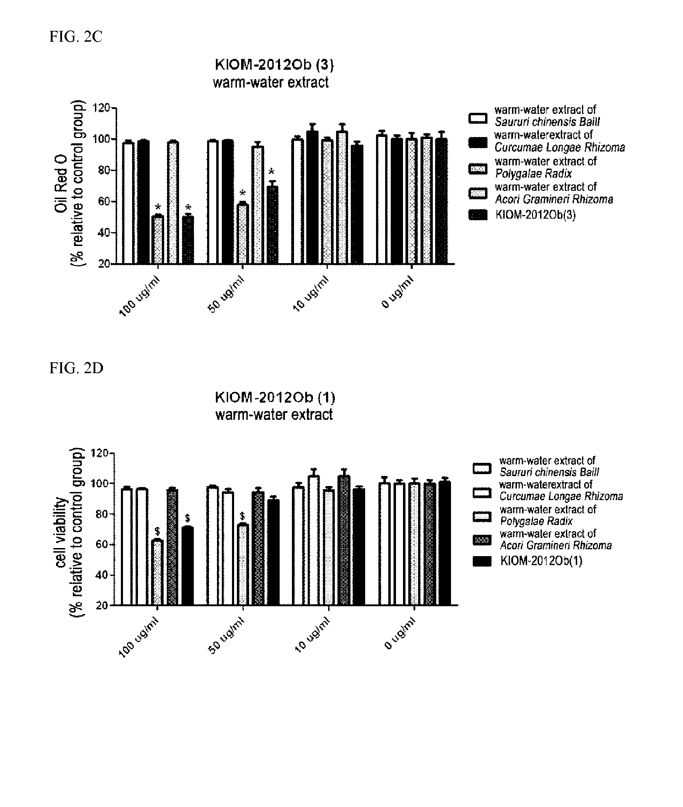

FIG. 2C is a graph illustrating the inhibitory effects of KIOM-2012Ob(3) warm-water extracts against the accumulation oftriglycerides within the adipocytes differentiated from 3T3-L1 via Oil Red O Staining. The Oil Red O Staining was performed 8 days after treating the cells with the KIOM-2012Ob(3) and each of the warm-water extracts of the herbal components constituted in the KIOM-2012Ob(3) while inducing differentiation from 3T3-L1. In the graph (FIG 2C), the treatment concentration was shown as the concentration of KIOM-2012Ob(3), and Saururi chinensis Baill., Curcumae Longae Rhizoma, Polygalae Radix, and Acori Gramineri Rhizoma constituted in KIOM-2012Ob(3) were treated in the same manner as in the contents of the composition. '*' denotes the significance (P<0.05) of the decrease in accumulation oftriglycerides relative to that of the control group (0 µg/mL).
FIG. 2D is a graph illustrating the effect of KIOM-2012Ob(1) warmwater extract on the viability of the cells differentiated from 3T3-L1. The viability of the cells differentiated from 3T3-L1 was shown after measurement via WST method. In the graph, the treatment concentration was shown as the concentration of KIOM-2012Ob(1), and Saururi chinensis Baill., Curcumae Longae Rhizoma, Polygalae Radix, and Acori Gramineri Rhizoma constituted in KIOM-2012Ob(1) were treated in the same manner as in the contents of the composition. '$' denotes the significance (P<0.05) of the decrease of the cell viability relative to that of the control group (0 µg/mL).

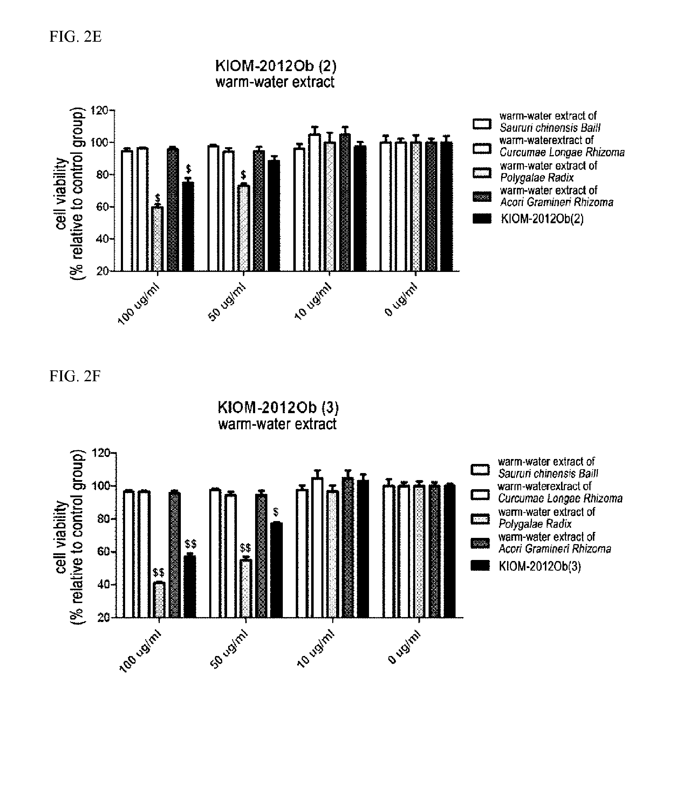

FIG. 2E is a graph illustrating the effect of KIOM-2012Ob(2) warm-water extract on the viability of the cells differentiated from 3T3-L1. The viability of the cells differentiated from 3T3-L1 was shown after measurement via WST method. In the graph, the treatment concentration was shown as the concentration of KIOM-2012Ob(2), and Saururi chinensis Baill., Curcumae Longae Rhizoma, Polygalae Radix, and Acori Gramineri Rhizoma constituted in KIOM-2012Ob(2) were treated in the same manner as in the contents of the composition. '$' denotes the significance (P<0.05) of the decrease of the cell viability relative to that of the control group (0 µg/mL).
FIG. 2F is a graph illustrating the effect of KIOM-2012Ob(3) warm-water extract on the viability of the cells differentiated from 3T3-L1. The viability of the cells differentiated from 3T3-L1 was shown after measurement via WST method. In the graph, the treatment concentration was shown as the concentration of KIOM-2012Ob(3), and Saururi chinensis Baill., Curcumae Longae Rhizoma, Polygalae Radix, and Acori Gramineri Rhizoma constituted in KIOM-2012Ob(3) were treated in the same manner as in the contents of the composition. '$' denotes the significance (P<0.05) of the decrease of the cell viability relative to that of the control group (0 µg/mL).

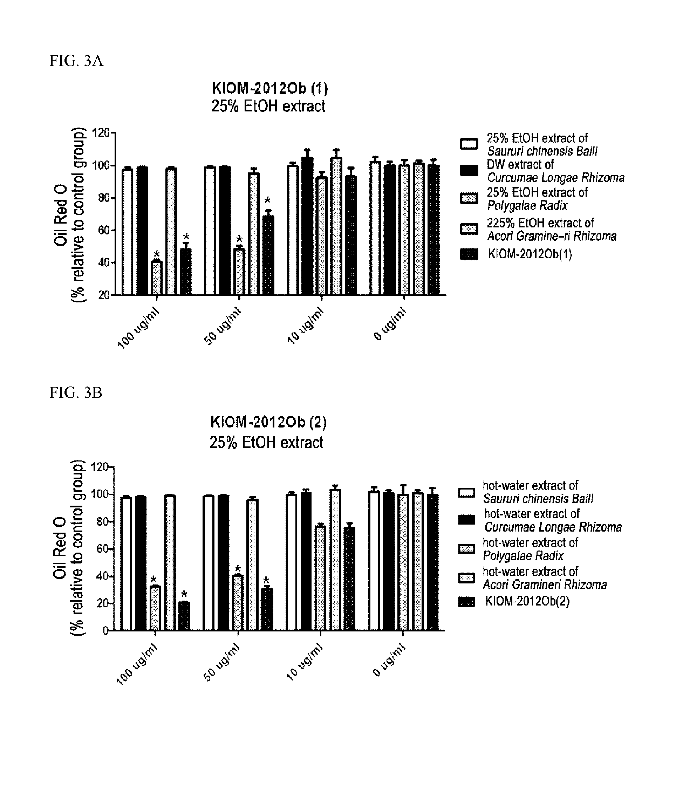

FIG. 3A is a graph illustrating the inhibitory effects of KIOM-2012Ob(1) 25% EtOH extracts against the accumulation of triglycerides within the adipocytes differentiated from 3T3-L1 via Oil Red O Staining. The Oil Red O Staining was performed 8 days after treating the cells with the KIOM-2012Ob(1) and each of the 25% EtOH extracts of the herbal components constituted in the KIOM-2012Ob(1) while inducing differentiation from 3T3-L1. In the graph, the treatment concentration was shown as the concentration of KIOM-2012Ob(1), and Saururi chinensis Baill., Curcumae Longae Rhizoma, Polygalae Radix, and Acori Gramineri Rhizoma constituted in KIOM-2012Ob(1) were treated in the same manner as in the contents of the composition. '*' denotes the significance (P<0.05) of the decrease in accumulation of triglycerides relative to that of the control group (0 µg/mL).
FIG. 3B is a graph illustrating the inhibitory effects of KIOM-2012Ob(2) 25% EtOH extracts against the accumulation of triglycerides within the adipocytes differentiated from 3T3-L1 via Oil Red O Staining. The Oil Red O Staining was performed 8 days after treating the cells with the KIOM-2012Ob(2) and each of the 25% EtOH extracts of the herbal components constituted in the KIOM-2012Ob(2) while inducing differentiation from 3T3-L1. In the graph, the treatment concentration was shown as the concentration of KIOM-2012Ob(2), and Saururi chinensis Baill., Curcumae Longae Rhizoma, Polygalae Radix, and Acori Gramineri Rhizoma constituted in KIOM-2012Ob(2) were treated in the same manner as in the contents of the composition. '*' denotes the significance (P<0.05) of the decrease in accumulation of triglycerides relative to that of the control group (0 µg/mL).

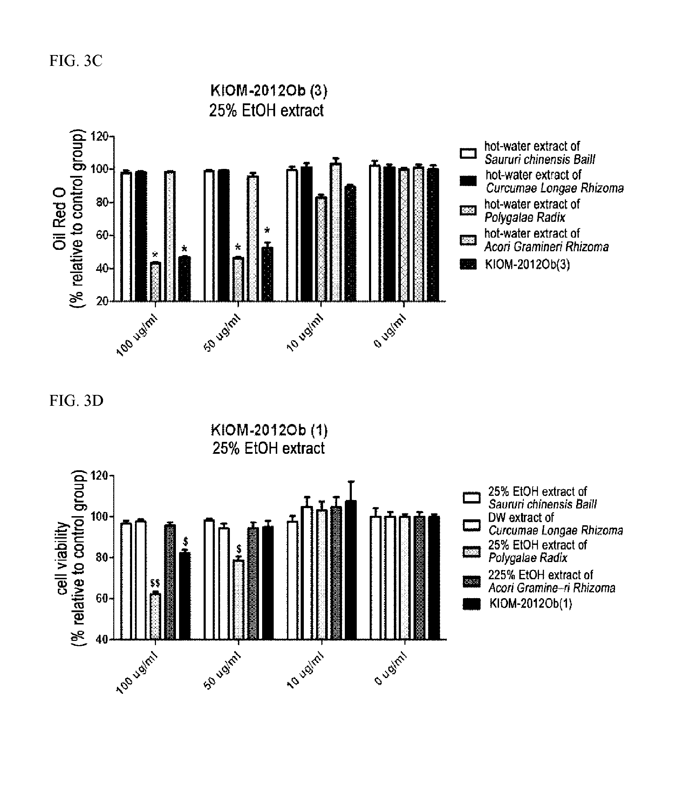

FIG. 3C is a graph illustrating the inhibitory effects of KIOM-2012Ob(3) 25% EtOH extracts against the accumulation oftriglycerides within the adipocytes differentiated from 3T3-L1 via Oil Red O Staining. The Oil Red O Staining was performed 8 days after treating the cells with the KIOM-2012Ob(3) and each of the 25% EtOH extracts of the herbal components constituted in the KIOM-2012Ob(3) while inducing differentiation from 3T3-L1. In the graph, the treatment concentration was shown as the concentration of KIOM-2012Ob(3), and Saururi chinensis Baill., Curcumae Longae Rhizoma, Polygalae Radix, and Acori Gramineri Rhizoma constituted in KIOM-2012Ob(3) were treated in the same manner as in the contents of the composition. '*' denotes the significance (P<0.05) of the decrease in accumulation of triglycerides relative to that of the control group (0 µg/mL).
FIG. 3D is a graph illustrating the effect of KIOM-2012Ob(1) 25% EtOH extract on the viability of the cells differentiated from 3T3-L1. The viability of the cells differentiated from 3T3-L1 was shown after measurement via WST method. In the graph, the treatment concentration was shown as the concentration of KIOM-2012Ob(1), and Saururi chinensis Baill., Curcumae Longae Rhizoma, Polygalae Radix, and Acori Gramineri Rhizoma constituted in KIOM-2012Ob(1) were treated in the same manner as in the contents of the composition. '$' denotes the significance (P<0.05) of the decrease of the cell viability relative to that of the control group (0 µg/mL). '$$' denotes the significance (*P*<0.01) of the decrease of the cell viability relative to that of the control group (0 µg/µL).
FIG. 3E is a graph illustrating the effect of KIOM-2012Ob(2) 25% EtOH extract on the viability of the cells differentiated from 3T3-L1. The viability of the cells differentiated from 3T3-L1 was shown after measurement via WST method. In the graph, the treatment concentration was shown as the concentration of KIOM-2012Ob(2), and Saururi chinensis Baill., Curcumae Longae Rhizoma, Polygalae Radix, and Acori Gramineri Rhizoma constituted in KIOM-2012Ob(2) were treated in the same manner as in the contents of the composition. '$' denotes the significance (P<0.05) of the decrease of the cell viability relative to that of the control group (0 µg/mL). '*' denotes the significance (*P*<0.05) of the increase of the cell viability by KIOM-2012Ob(2) 25% EtOH extract relative to that by Polygalae Radix 25% EtOH extract.
FIG. 3F is a graph illustrating the effect of KIOM-2012Ob(3) 25% EtOH extract on the viability of the cells differentiated from 3T3-L1. The viability of the cells differentiated from 3T3-L1 was shown after measurement via WST method. In the graph, the treatment concentration was shown as the concentration of KIOM-2012Ob(3), and Saururi chinensis Baill., Curcumae Longae Rhizoma, Polygalae Radix, and Acori Gramineri Rhizoma constituted in KIOM-2012Ob(3) were treated in the same manner as in the contents of the composition. '$' denotes the significance (P<0.05) of the decrease of the cell viability relative to that of the control group (0 µg/mL). '$$' denotes the significance (P<0.01) of the decrease of the cell viability relative to that of the control group (0 µg/mL). '*' denotes the significance (*P*<0.05) of the increase of the cell viability by KIOM-2012Ob(3) 25% EtOH extract relative to that by Polygalae Radix 25% EtOH extract.
FIG. 4 is a graph illustrating the effect of KIOM-2012Ob 25% EtOH extracts on the decrease of body weight in an obesity animal model. A normal control group fed a diet with only 10% fat content, a negative control group fed a diet with only 60% fat content (HFD), and a positive control group fed with [Xenical (62.5 mg/kg) + diet with 60% fat content] were used. In the graph, '1X' indicates the dose of the extract for an adult male based on the daily administration of the herbal decoction, and '4X' indicates four times the 1X dose. The graph shows a distinct decrease in body weight in KIOM-2012Ob-4X groups.
FIG. 5 is a graph illustrating the amount of dietary intake of the mice treated with KIOM-2012Ob 25% EtOH extracts. '*' denotes the significance (*P*<0.05) of the decrease in the amount of dietary intake in mice treated with KIOM-2012Ob(3) EtOH extract relative to the amount of dietary intake of mice fed with 60% HFD.
FIG. 6 is a graph illustrating the effect of KIOM-2012Ob 25% EtOH extracts on the fat accumulation. A normal control group fed a diet with only 10% fat content, a negative control group fed a diet with only 60% fat content (HFD), and a positive control group fed with [Xenical (62.5 mg/kg) + diet with 60% fat content] were used. In the graph, '1X' indicates the dose of the extract for an adult male based on daily administration of the herbal decoction and '4X' indicates four times the '1X' dose. For the weight of the fat tissues of the mice, their abdominal fat and subcutaneous fat were measured. '**' denotes the significance (P<0.05) of the decrease in the weight of abdominal fat of the mice treated with KIOM-2012Ob(3) EtOH extract relative to the body weight of the mice fed with 60% HFD.
FIG. 7 is a graph illustrating the effect ofthe KIOM-2012Ob 25%EtOH extracts on the liver. The liver tissues were fixed on formalin and their H & E Staining and pathological findings were requested, wherein FIG. 7A shows the increase of microvesicular steatosis in the liver tissues, and the inhibition of the increase of the microvesicular steatosis in the group treated with KIOM-2012Ob, whereas FIG. 7B shows the increase of chronic inflammation inliver tissues. The group treated with KIOM-2012Ob showed a significant decrease in inflammation (P<0.05, P<0.01) than the groups treated with other herbal drugs, in particular the group treated with Polygalae Radix extract. '$$' denotes the significance (*P*<0.01) of the increase of microvesicular steatosis relative to that of the normal control group, whereas '$' denotes the significance (P<0.05) of the increase of microvesicular steatosis relative to that of the normal control group. '**' denotes the significance (P<0.01) of the decrease of chronic inflammation in the group treated with KIOM-2012Ob relative to the group treated with Polygalae Radix extract (4X), whereas '*' denotes the significance (P<0.05) of the decrease of chronic inflammation in the group treated with KIOM-2012Ob relative to the group treated with Polygalae Radix extract (4X). '##' denotes the significance (P<0.01) of the increase of chronic inflammation relative to the negative control group, whereas '#' denotes the significance (P<0.05) of the increase of chronic inflammation relative to the negative control group.
FIG. 8 is a graph illustrating the effect of the KIOM-2012Ob 25% EtOH extracts on the kidney. The kidney tissues were fixed on formalin and their H & E Staining and pathological findings were requested, wherein FIG. 7A shows the increase of microvesicular steatosis in the liver tissues, and the inhibition of the increase of the microvesicular steatosis in the group treated with KIOM-2012Ob, whereas FIG. 7B shows the increase of chronic inflammation in the liver tissues. The graph shows an increase of chromic inflammation in the kidney. The group treated with KIOM-2012Ob showed a significant decrease in inflammation (P<0.05, P<0.01) than the groups treated with other herbal drugs, and in particular, the group treated with Polygalae Radix extract. '**' denotes the significance (P<0.01) of the decrease of chronic inflammation in the group treated with KIOM-2012Ob relative to the group treated with Polygalae Radix extract (4X), whereas '*' denotes the significance (P<0.05) of the decrease of chronic inflammation in a group treated with KIOM-2012Ob relative to the group treated with Polygalae Radix extract (4X). '###' denotes the significance (P<0.001) of the increase of chronic inflammation relative to the negative control group, whereas '##' denotes the significance (P<0.01) of the increase of chronic inflammation relative to the negative control group, and '#' denotes the significance (P<0.05) of the increase of chronic inflammation relative to the negative control group.
FIG. 9 is a graph illustrating the comparison result of the body weight of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 10 is a graph illustrating the comparison result of the amount of dietary intake of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 11A is a graph illustrating the comparison result of the weight of abdominal gonadal fat of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 11B is a graph illustrating the comparison result of the weight of mesenteric fat of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 11C is a graph illustrating the comparison result of the weight of subcutaneous fat of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 11D is a graph illustrating the comparison result of the total fat of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 12A is a graph illustrating the comparison result of the level of activated Ghrelin of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 12B is a graph illustrating the comparison result of the level of activated desacyl-ghrelin of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 12C is a graph illustrating the comparison result of the level of activated ghrelin ratio of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 13 is a graph illustrating the comparison result of the level of leptin in the blood plasma of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 14A is a graph illustrating the comparison result of the level of triglycerides in the blood of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 14B is a graph illustrating the comparison result of the level of total cholesterol in the blood of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 14C is a graph illustrating the comparison result of the level of LDL-cholesterol in the blood of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 14D is a graph illustrating the comparison result of the level ofHDL-cholesterol in the blood of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 14E is a graph illustrating the comparison result of the level of glucose in the blood of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 14F is a graph illustrating the comparison result of the level of creatinine in the blood of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 14G is a graph illustrating the comparison result of the level ofurea in the blood of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 14H is a graph illustrating the comparison result of the level of LDH (Lactate dehydrogenase) in the blood of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 14I is a graph illustrating the comparison result of the level of ALP (alkaline phosphatase) in the blood of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 14J is a graph illustrating the comparison result of the level of GPT (glutamic-pyruvic transaminase) in the blood of an animal model according to the administered dose of an extract of a mixture comprising Saururi chinensis Baill., Curcumae Longae Rhizoma, and Polygalae Radix, and selectively comprising Acori Gramineri Rhizoma or clover.
FIG. 14K is a graph illustrating the comparison result of the level of GOT (glutamic-oxaloacetic transaminase) in the blood of an animal model according to the administered dose of an extract of a mixture comprising Saururi chinensis Baill., Curcumae Longae Rhizoma, and Polygalae Radix, and selectively comprising Acori Gramineri Rhizoma or clover.

### DESCRIPTION OF THE PREFERRED EMBODIMENTS

Features and advantages of the present invention will be more clearly understood by the following detailed description of the present preferred embodiments by reference to the accompanying drawings. It is first noted that terms or words used herein should be construed as meanings or concepts corresponding with the technical sprit of the present invention, based on the principle that the inventor can appropriately define the concepts of the terms to best describe his own invention. Furthermore, it should be understood that detailed descriptions of well-known functions and structures related to the present invention will be omitted so as not to unnecessarily obscure the important point of the present invention.

As used herein, the term "Saururi chinensis Baill." refers to a perennial plant belonging to the Saururaceae Family, Piperales Order, which has been used as herbal raw ingredients in herbal medicines. Saururi chinensis Baill. is known to have a bitter and spicy tastes, white flowers and roots, the top two or three leaves turn white when in bloom, and bears a single round fruit in each flower bud between September and October in the northern hemisphere.

In the present invention, Saururi chinensis Baill. may be used as an active ingredient of a pharmaceutical composition for the prevention or treatment of obesity; The parts used are leaves, stems, roots or the entire plant.

As used herein, the term "Curcumae Longae Rhizoma" refers to a perennial plant belonging to Zingiberaceae Family, Zingiberales Order, which has been used as herbal raw ingredients in herbal medicines. Curcumae Longae Rhizoma is known to have bitter and spicy tastes, the surface of its rhizome takes on a light yellow while its inner side is vermillion and emits a camphor-like aroma, its flower pettles come out before the leaves, and its yellow flowers bloom from the axils of leaves between April and June in the northern hemisphere.

In the present invention, Curcumae Longae Rhizoma may be used as an active ingredient of a pharmaceutical composition for the prevention or treatment of obesity, and the parts used are stems or roots of the plant.

As used herein, the term "Polygalae Radix" refers to a perennial plant belonging to the Polygalaceae Family, Geraniales Order, which has been used as herbal raw ingredients in herbal medicines. Polygalae Radix is known that it has bitter and spicy tastes, long and thick roots with a ridge but almost no hairs except some slightly curled hairs on the upper part, and its flowers bloom in violet between July and August in the nothern hemissphere, and its fruits have many seeds contained in the partitioned space therein.

In the present invention, Polygalae Radix may be used as an active ingredient of a pharmaceutical composition for the prevention or treatment of obesity, and the parts used are roots of the plant.

As used herein, the term "Acori Gramineri Rhizoma" refers to a perennial plant belonging to the Araceae Family, Arales Order, which has been used as herbal raw ingredients in herbal medicines. Acori Gramineri Rhizoma is known to have laterally extending rhizomes, fibrous roots coming out of its joints, wherein the space between joints is relatively shorter above ground while that under ground is longer, is green overall, have a hermaphrodite flower including both pistils and stamens, its flowers bloom between June and July in yellow, and its fruits have many seeds contained in the partitioned space therein.

In the present invention, Polygalae Radix may be used as an active ingredient of a pharmaceutical composition for the prevention or treatment of obesity, and the parts used are stems and roots of the plant.

As used herein, the term "extract of a mixture" refers to an extract of a mixture by extracting a mixture comprising Saururi chinensis Baill., Curcumae Longae Rhizoma and Polygalae Radix, or a mixture comprising Saururi chinensis Baill., Curcumae Longae Rhizoma, Polygalae Radix and Acori Gramineri Rhizoma.

Depending on the plant part(s) for extraction, the activities of each of the extract(s), fraction(s) thereof, and the specific ingredients contained therein may differ from each other. Accordingly, the present inventors firstly identified that an extract of a mixture comprising the entire plant of Saururi chinensis Baill., stems and roots of Curcumae Longae Rhizoma, and roots of Polygalae Radix; and an extract of a mixture comprising the entire plant of Saururi chinensis Baill., stems and roots of Curcumae Longae Rhizoma, roots of Polygalae Radix, and stems and roots of Acori Gramineri Rhizoma exhibit the effect of preventing or treating obesity.

In the present invention, the extract may be used as an active ingredient of a pharmaceutical composition for preventing or treating obesity. The amounts of Saururi chinensis Baill., Curcumae Longae Rhizoma, and Polygalae Radix included in the mixture for obtaining the extract are not particularly limited as long as they can be used in manufacturing a composition for preventing or treating obesity. Preferably, Saururi chinensis Baill., Curcumae Longae Rhizoma, and Polygalae Radix may be mixed at a ratio of 1 : 0.2 through 1 : 0.2 through 1 (w/w/w), more preferably, 1 : 0.4 through 1 : 0.4 through 1 (w/w/w), and most preferably, 1 : 0.6 : 1 (w/w/w).

Additionally, when an extract of a mixture is obtained from the mixture including Saururi chinensis Baill., Curcumae Longae Rhizoma, Polygalae Radix, and Acori Gramineri Rhizoma, the amounts of Saururi chinensis Baill., Curcumae Longae Rhizoma, Polygalae Radix, and Acori Gramineri Rhizoma are not particularly limited as long as they can be used in manufacturing a composition for the prevention or treatment of obesity. Preferably, Saururi chinensis Baill., Curcumae Longae Rhizoma, Polygalae Radix, and Acori Gramineri Rhizoma may be mixed at a ratio of 1 : 0.2 through 1 : 0.2 through 1 : 0.2 through 1 (w/w/w/w), more preferably, 1 : 0.4 through 1 : 0.4 through 1 : 0.4 through 1 (w/w/w/w), and most preferably, 1 : 0.6 : 1 : 0.4 (w/w/w/w). Additionally, Saururi chinensis Baill., Curcumae Longae Rhizoma, Polygalae Radix, and Acori Gramineri Rhizoma may be purchased from the market or those cultivated in nature may be used.

Examples of the extraction methods may include, although not limited thereto, preferably boiled-water extraction, hot-water extraction, cold immersion extraction, reflux cooling extraction, or ultrasonification extraction, etc.

The extract may be obtained by extracting using an extraction solvent, or by fractionating the extract obtained by extracting using an extraction solvent. The extraction solvent may include, although not limited thereto, water, an organic solvent, or a mixed solvent thereof. The organic solvent may include a C₁ ∼ C₄ alcohol, a polar solvent such as ethyl acetate or acetone, a non-polar solvent such as hexane or dichloromethane, or a mixed solvent thereof Additionally, preferably, water, a C₁ ∼ C₄ alcohol, or a mixed solvent thereof may be used, more preferably, ethanol. In an exemplary embodiment of the present invention, the extract was obtained using 25% ethanol (EtOH) as the solvent.

The extract may be contained in an amount from 0.001 wt% to 100 wt% relative to the total weight of the respective pharmaceutical composition, more preferably 0.1 wt% to 80 wt%.

As used herein, the term "fraction" refers to a resultant product obtained by the fractionation method for separating a specific component or specific group from the respective extract.

In the present invention, the fraction may be obtained via various methods for fractionating the extract, and may include solvent fractionation performed by treating various solvents, ultrafiltration fractionation performed by passing through an ultrafiltration membrane having a certain molecular cut-off value, chromatography fractionation performed by various chromatographies (manufactured for separation according to size, electric charge, hydrophobicity, or affinity), etc. , although not limited thereto. In particular, the solvent to be used in the solvent fractionation may be a polar or non-polar solvent, although not limited thereto, and preferably a non-polar solvent. The solvent fractionation may be performed by fractionating the extract using the fractionation solvent in a sequential order from a higher non-polarity solvent to a lower non-polarity solvent. For example, the extract may be sequentially fractionated using hexane or ethyl acetate.

The fraction may be contained in an amount from 0.001 wt% to 100 wt% relative to the total weight of the respective pharmaceutical composition, more preferably 0.1 wt% to 80 wt%.

As used herein, the term "pharmaceutical composition" refers to something manufactured for the purpose of preventing or treating a disease(s), and may be prepared in various types of formulations according to the corresponding conventional method. For example, it may be prepared in oral-type formulations such as powders, granules, tablets, capsules, suspensions, and syrups, and also used in the form of external formulations, suppositories, and sterile injection solutions.

As used herein, the term "obesity" refers to an abnormal health condition caused by the increase of body weight, in which, due to an excess energy intake over energy consumption, the unconsumed surplus energy can accumulate as body fat in various forms of lipids or a physical state with an elevated lipid content in the blood. Once obesity occurs, it may induce various metabolic diseases and adult diseases such as diabetes, hyperlipidemia, heart diseases, strokes, atherosclerosis, and fatty liver.

The composition provided in the present invention can not only treat obesity by inhibiting fat production, but also improve, prevent or treat obesity-related diseases derived from obesity, for example, various metabolic diseases such as diabetes, hyperlipidemia, heart diseases, strokes, atherosclerosis, and fatty liver.

As used herein, the term "prevention" refers to all kinds of activities associated with the inhibition or delay of obesity or obesity-related diseaseas by administering the compositon of the present invention.

As used herein, the term "treatment" refers to all kinds of actions associated with the improvement or advantageous changes in symptoms of obesity or obesity-related diseaseas

In an exemplary embodiment of the present invention, hot-water extracts (an extract of a mixture and individual extracts), warm-water extracts (an extract of a mixture and individual extracts), and ethanol extracts (an extract of a mixture and individual extracts) of a mixture including Saururi chinensis Baill., Curcumae Longae Rhizoma, Polygalae Radix and Acori Gramineri Rhizoma, and a mixture including each of them separately were obtained, respectively (Table 1), and 3T3-L1 cells were treated with each of the extracts at varied concentrations (Table 2) and their *in vitro* anti-obesity effects were examined. As a result, it was confirmed that, among the hot-water extracts (FIGS. 1A through 1C), warm-water extracts (FIGS. 2A through 2C), and ethanol extracts (FIGS. 3A through 3C), the extracts of a mixture and Polygalae Radix extracts showed the effects of reducing fat content and the number of the cells, whereas Saururi chinensis Baill. extracts, Curcumae Longae Rhizoma extracts and Acori Gramineri Rhizoma extracts showed no reducing effects of fat content and the number of the cells at all. Additionally, among the hot-water extracts, the warm-water extracts, and the ethanol extracts, the ethanol extracts were shown to have the most superior effects of reducing fat contents and safety. Additionally, high fat diet containing each of the extracts at varied concentrations was administered to mice (Table 3), and their *in vivo* anti-obesity effects were examined. As a result, it was shown that, the mice treated with Polygalae Radix extracts and the extracts of a mixture showed the most superior effect of reducing body weight, and of them in particular, the Polygalae Radix extracts showed severe toxicities, whereas the extract of a mixture (ethanol extracts) showed alleviated toxicities (FIG. 4). Additionally, the measurement of fat weight revealed that the Polygalae Radix extracts exhibited the most significant inhibitory effect against fat increase, followed by the Curcumae Longae Rhizoma extracts, and the Acori Gramineri Rhizoma extracts showed the least inhibitory effect against fat increase (FIG. 6); and the extract of a mixture (ethanol extracts) showed higher safety than the Polygalae Radix extracts in liver tissues (FIG. 7) and kidney tissues (FIG. 8).

In another exemplary embodiment of the present invention, in order to examine the inhibitory effects of the extract of a mixture including Saururi chinensis Baill., Curcumae Longae Rhizoma and Polygalae Radix against obesity, exclusive of Acori Gramineri Rhizoma, which was analyzed to have the least inhibitory effect against fat increase among the components included in the extract of a mixture, ethanol extracts were obtained for each of the components from the mixture essentially including Saururi chinensis Baill., Curcumae Longae Rhizoma, and Polygalae Radix, and selectively including Acori Gramineri Rhizoma and clover, respectively (Table 4). Each of the thus-obtained ethanol extracts was administered to mice at varied doses, and the experimental animals were collected thereafter (Table 5) and subjected to evaluations regarding their body weight (FIG. 9), dietary intake (FIG. 10), fat weight (FIGS. 11A through 11D), ghrelin level (FIGS. 12A through 12C), leptin level (FIG. 13), and levels of components in blood (FIGS. 14A through 14K). As a result, it was confirmed that not only the extract of a mixture including Saururi chinensis Baill., Curcumae Longae Rhizoma, and Polygalae Radix but also the extract of a mixture, which additionally includes Acori Gramineri Rhizoma thereto, exhibited improved anti-obesity effects compared to that of the control group.

Accordingly, it was confirmed that the extract of a mixture of the anti-obesity composition may be obtained from a mixture essentially including Saururi chinensis Baill., Curcumae Longae Rhizoma, and Polygalae Radix, or a mixture additionally including Acori Gramineri Rhizoma thereto.

The pharmaceutical composition of the present invention may further include a pharmaceutically acceptable diluent, excipient or carrier. The pharmaceutical composition including the pharmaceutically acceptable carrier may be prepared in various formulations for oral or parenteral administration. Formulations may be prepared using a conventional filler, binder, humectant, disintegrant, diluent, surfactant or excipient. Examples of the solid formulations for oral administration may include tablets, pills, powders, granules, capsules, etc., and the solid formulations may include at least one excipient, e.g., starch, calcium carbonate, sucrose or lactose, gelatin, etc., to be mixed therein. Additionally, lubricants such as magnesium stearate and talc may be also used in addition to the simple excipient. Examples of the liquid formulations for oral administration may include suspensions, liquid medicines for internal use, emulsifiers, syrups, etc., which may include various kinds of excipients, e.g., a humectants, a sweetener, a fragrant, a preservative, etc., in addition to the commonly used simple diluents such as water and liquid paraffin. Examples of the formulations for parenteral administration may include sterile aqueous solutions, non-aqueous solvents, suspensions, emulsifiers, lyophilized formulations, and suppositories. Examples of the non-aqueous solvents and suspensions may include propylene glycol, polyethylene glycol, a vegetable oil such as olive oil, injectable estersuch as ethyl oleate, etc. Examples of the bases for the suppositories may include witepsol, macrogol, tween 61, cacao butter, laurinum, glycerogelatin, etc.

The pharmaceutical composition may be prepared in any one of the formulations selected from the group consisting of tablets, pills, powders, granules, capsules, suspensions, liquid medicines for internal use, emulsifiers, syrups, sterile aqueous solutions, non-aqueous solvents, suspensions, emulsifiers, lyophilized formulations and suppositories.

In another aspect of the present invention to accomplish the objectives, there is provided a method for the prevention or treatment of obesity including administering an effective amount of a pharmaceutical composition comprising the extract of a mixture or a fraction thereof as an active ingredient to a subject having a risk of obesity or a subject having obesity. In particular, the respective active ingredient to be contained in the extract of a mixture is the same as described above.

As used herein, the term "subject" refers to all kinds of animals including humans having a risk of obesity or having obesity.

As used herein, the term "administration" refers to an activity of introducing the pharmaceutical composition of the present invention to a subject by an appropriate method.

In the present invention, the pharmaceutical composition may be administered via any routes as long as they can deliver the same to the target tissues. According to the intended purposes, the pharmaceutical composition of the present invention may be administered via oral administration, rectal administration, local administration, intraperitoneal administration, intravenous administration, intraarterial administration, intramuscular administration, intranasal administration, subcutaneous administration, intracutaneous administration, dermal administration, intranasal administration, intrapulmonary administration, intrarectal administration, ocular administration, etc., but is not limited thereto. Additionally, the pharmaceutical composition may be administered via any apparatus that enables to transport the the active ingredient to a target cell.

In an exemplary embodiment, the pharmaceutical composition of the present invention includes an extract of a mixture. The amount of the extract of a mixture contained in the composition may be in the range from 0.1 wt% to 50 wt% relative to the total weight of the pharmaceutical composition, although not limited thereto.

The pharmaceutical composition of the present invention may be administered in a pharmaceutically effective amount, and as used herein, the term "pharmaceutically effective amount" refers to an amount sufficient to sufficient for the treatment of diseases at a reasonable benefit/risk ratio applicable to a medical treatment without causing any adverse effects, and the level of the effective dose may be determined factors including the kind of a subject, severity of illness, age, sex, drug activity, drug sensitivity, administration time, administration route and dissolution rate, length of treatment, drug(s) used in combination, and other factors well known in the medical field. The pharmaceutical composition of the present invention may be administered as an individual therapeutic agent or in combination with other therapeutic agent, and also sequentially or simultaneously with the conventional therapeutic agent. Additionally, the pharmaceutical composition of the present invention may be administered as a single dose or in multiple divided doses. Additionally, it is important that an amount which can achieve the maximum effect with the least amount without any adverse effects be administered in consideration of all the factors described above.

The dose of the pharmaceutical composition of the present invention may be determined by a skilled person in the art considering the intended use(s), addiction level of disease(s), age, body weight, sex and anamesis of a subject, or the kinds of ingredients usd as active ingredient(s), etc. In an exemplary embodiment, the pharmaceutical composition of the present invention may be administered in the range from about 1 µg/kg/day to about 100 mg/kg/day for an adult, preferably from 20 mg/kg/day to 30 mg/kg/day. The pharmaceutical composition of the present invention may be administered once daily or in a few divided doses, although not particularly limited thereto.

In still another aspect of the present invention to achieve the objectives, there is provided a food composition for the prevention or improvement of obesity comprising the extract or a fraction thereof

The Saururi chinensis Baill., Curcumae Longae Rhizoma, Polygalae Radix and Acori Gramineri Rhizoma included in the extract of a mixture have long been used as herbal raw ingredients and their safety has been approved accordingly. Therefore, they may be prepared in the form of foods for preventing or treating obesity for ingestion. In particular, the extract of a mixture or a fraction thereof may be included in the range from 0.01 wt% to 100 wt% relative to the total weight of the food composition, preferably from 1 wt% to 80 wt%, although not limited thereto. When the food is beverage it may be included in the range from 1 g to 30 g per 100 mL of the food composition, preferably from 3 g to 20 g. Additionally, the composition may further include additional ingredients which may be conventionally used in food compositions to improve smell, taste, sight, etc., e.g., vitamins A, C, D, E, B1, B2, B6, B12, niacin, biotin, folate, panthotenic acid, etc. Additionally, the composition may further include minerals such as Zn, Fe, Ca, Cr, Mg, Mn, Cu, etc. Additionally, the composition may further include amino acids such as lysine, tryptophan, cysteine, valine, etc. Addtionally, the composition may further include food additives such as preservatives (potassium sorbate, sodium benzoate, salicylic acid, dehydro sodium acetate, etc.), disinfectants (bleaching powder, higher bleaching powder, sodium hypochlorite, etc.), antioxidants (butylhydroxyanisole (BHA), butylhydroxytoluene (BHT), etc.), coloring agents (tar color, etc.), color-developing agents (sodium nitrite, etc.), bleaching agents (sodium sulfite), seasonings (monosodium glutamate (MSG), etc.), sweeteners (dulcin, cyclemate, saccharin, sodium, etc.), flavors (vaniline, lactones, etc.), swelling agents (alum, potassium D-hydrogn tartate, etc.), fortifiers, emulsifiers, thickners (adhesive pastes), film-forming agents, gum base agents, antifoaming agents, solvents, improvers, etc. The food additives may be selected according to the food kinds and used in an appropriate amount.

Additionally, functional foods for preventing or improving obesity may be manufactured using a food compositin comprising the extract or a fraction thereof

Specifically, processed foods for preventing or improving obesity may be manufactured using the food composition. Examples of the processed foods may include cookies, beverages, alcoholic beverages, fermented foods, canned foods, milk-processed foods, meat-processed foods, noodles, etc. Examples of the cookies include biscuits, pies, cakes, breads, candies, jellies, gums, cereals (meal substitutes such as grain flakes). Examples of the beverages include drinking water, carbonated soft drinks, functional isotonic drinks, juices (e.g., apple-, pear-, grape-, aloe-, tangerine-, peach-, carrot-, tomato juices, etc.), sweet rice drinks, etc. Examples of the alcoholic beverages include refined rice wine, whisky, beer, liquors, fruits wine, etc. Examples of the fermented foods include soy sauce, bean paste, red pepper paste, etc. Examples of the canned foods include seafood canned foods (e.g., canned tuna, mackerel, mackerel pike, conch, etc.), livestock canned foods (canned beef, pork, chicken, turkey, etc.), agricultural canned foods (canned corn, peach, pineapple, etc.). Examples of milk-processed foods include cheese, butter, yogurt, etc. Examples of meat-processed foods include pork cutlets, beef cutlets, chicken cutlets, sausages, sweet and sour porks, nuggets, neobiani, etc. Examples of the noodles include sealed and packed fresh noodles. Additionally, the composition may be used for manufacturing retort foods, soups, etc.

As used herein, the term "functional food", which has the same meaning as the term "for special health use (FoSHU)", refers to a food with high effects of medicinal and medical treatment modulated so as to efficiently exhibit body modulating function as well as provision of nutrients. The functional food may be manufactured in various forms including tablets, capsules, powders, granules, liquids, pills, etc., in order to obtain useful effects for the prevention or improvement of bone diseases.

In still another aspect of the present invention to achieve the objectives, there is provided a method for manufacturing the extract of a mixture including obtaining the extract from a mixture including Saururi chinensis Baill., Curcumae Longae Rhizoma, and Polygalae Radix using water, a C₁ ∼ C₄ alcohol, or a mixed solvent thereof

In the method for manufacturing the extract of a mixture, the method may further include a conventional extraction method used in the art such as hot-water extraction, warm-water extraction, ethanol extraction, ultrasonification extraction, filtration and reflux extraction, and after obtaining the extract, the extract may be further subjected to filtration, concentration, or lyophilization.

In particular, each of the active ingredients included in the extract of a mixture is the same as described above.

The mixture may include Saururi chinensis Baill., Curcumae Longae Rhizoma and Polygalae Radix mixed therein at a ratio of 1 : 0.2 through 1 : 0.2 through 1 (w/w/w).

In still another aspect of the present invention to achieve the objectives, there is provided a method for manufacturing an extract of a mixture comprising Saururi chinensis Baill., Curcumae Longae Rhizoma, Polygalae Radix, and Acori Gramineri Rhizoma, which includes obtaining the extract from a mixture including Saururi chinensis Baill., Curcumae Longae Rhizoma, Polygalae Radix and Acori Gramineri Rhizoma using water, a C₁ ∼ C₄ alcohol, or a mixed solvent thereof In particular, each of the active ingredients included in the extract of a mixture is the same as described above.

The mixture may include Saururi chinensis Baill., Curcumae Longae Rhizoma, Polygalae Radix and Acori Gramineri Rhizoma mixed therein at a ratio of 1 : 0.2 through 1 : 0.2 through 1: 0.2 through 1 (w/w/w/w).

In still another aspect of the present invention to achieve the objectives, there is provided a composition including an extract of a mixture comprising Saururi chinensis Baill., Curcumae Longae Rhizoma, and Polygalae Radix or a fraction thereof. In particular, each of the active ingredients included in the extract of a mixture is the same as described above.

The composition may be used for treating obesity.

The extract of a mixture may be an extract of a mixture, in which Saururi chinensis Baill., Curcumae Longae Rhizoma, and Polygalae Radix are mixed therein at a ratio of 1 : 0.2 through 1 : 0.2 through 1 (w/w/w).

The extract of a mixture may be obtained using 25% ethanol.

In still another aspect of the present invention to achieve the objectives, there is provided a composition including an extract of a mixture comprising Saururi chinensis Baill., Curcumae Longae Rhizoma, Polygalae Radix, and Acori Gramineri Rhizoma or a fraction thereof. In particular, each of the active ingredients included in the extract of a mixture is the same as described above.

The composition may be used for treating obesity.

The extract of a mixture may be an extract of a mixture, in which Saururi chinensis Baill., Curcumae Longae Rhizoma, Polygalae Radix, and Acori Gramineri Rhizoma are mixed therein at a ratio of 1 : 0.2 through 1 : 0.2 through 1 : 0.2 through 1 (w/w/w).

The extract of a mixture may be obtained by extracting the mixture with 25% ethanol.

### ADVANTAGEOUS EFFECT OF THE INVENTION

The extract of a mixture of the present invention can inhibit fat production without the side effect of cell toxicity, and thus can be widely used for the prevention, improvement, or treatment of obesity or obesity-related diseases.

The present invention will be explained in greater detail through the following examples as set forth herein below, but they are disclosed for illustrative purposes only and are not to be construed as limiting the scope of the present invention.

### Example 1: Preparation of Individual Extracts of Saururi chinensis Baill., Curcumae Longae Rhizoma, Polygalae Radix, and Acori Gramineri Rhizoma, and extract of a mixture thereof

Saururi chinensis Baill., Curcumae Longae Rhizoma, Polygalae Radix, and Acori Gramineri Rhizoma were respectively ground and mixed according to the ratio shown in Table 1 below to prepare 30 g mixtures. The mixtures were then subjected to hot-water extraction, warm-water extraction, and ethanol extraction, respectively, and obtained the respective extract therefrom. Each of the thus-obtained extracts was subjected to filtration to obtain a liquid component, and the liquid component was lyophilized to obain powdered extracts (hot-water extract, warm-water extract, and ethanol extract), which were then dissolved in distilled water at a concentration of 100 mg/mL to be used later as samples for experiments. Specifically, the hot-water extraction was performed by adding 1 L of drinking water to the mixture, followed by heating to a final volume of 100 mL. The warm-water extraction was performed for 24 hours after adding 300 mL of distilled water to the mixture while maintaining the temperature at 38°C. The ethanol extraction was performed for 24 hours after adding 300 mL of 25% ethanol to the mixture while maintaining the temperature at 38°C.

Additionally, each 30 g of Saururi chinensis Baill., Curcumae Longae Rhizoma, Polygalae Radix, and Acori Gramineri Rhizoma was subjected to the same method as described above, and the individual extracts for each component (hot-water extract, warm-water extract, and ethanol extract) were prepared therefrom

**[Table 1] Composition of Extracts**

| **Extract** | **Saururi chinensisBaill.** (%) | **Curcumae Longae Rhizoma** (%) | **Polygalae Radix** (%) | **Acori Gramineri Rhizoma** (%) | **Total** (%) |
|---|---|---|---|---|---|
| KIOM-2012Ob(1) | 10g (33.3) | 10g (33.3) | 6g (20) | 4g (13.4) | 30g (100) |
| KIOM-2012Ob(2) | 10g (33.3) | 6g (20) | 10g (33.3) | 4g (13.4) | 30g (100) |
| KIOM-2012Ob(3) | 10g (33.3) | 4g (13.4) | 10g (33.3) | 6g (20) | 30g (100) |

### Example 2: In Vtro Anti-obesity Effects of Individual Extracts of Saururi chinensis Baill., Curcumae Longae Rhizoma, Polygalae Radix, and Acori Gramineri Rhizoma and extract of a mixture

3T3-L1 cells distributed by American Type Culture Collection (ATCC, USA) were inoculated into a Dulbcco's Modified Eagle's Medium (DMEM, Lonza) containing 10% bovine serum (BS; Gibco) and 1% penicillin-streptomycin, cultured at 37°C under 5% CO₂, subcultured at intervals of from 3 to 4 days and thereby obtained preadipocytes.

The thus-obtained preadipocytes were collected, adjusted to a concentration of 1 x 10⁴ cells/mL, aliquoted into a 96-well plate in the amount of 100 µL/well, and cultured at 37°C under 5% CO₂ for 4 days until they were saturated. Then, the thus-cultured cells were added with a medium for inducing differentiation of preadipocytes containing the individual extracts and the extract ofthe mixture (DMEM, 0.25 µM dexamethansone, 0.5 mM 3-isobutyl-1-methylxanthine, 10 µg/mL insulin, and fetal bovine serum (FBS)) and cultured further for 2 days. In particular, the concentrations of the individual extracts and the extract of the mixture included in the medium for inducing differentiation of preadipocytes are shown in Table 2 below.

**[Table 2] Treated Concentrations of Individual Extracts and Extract of the mixture**

| **Extract** | **Treated Concentration** (µg/mL) | | | |
|---|---|---|---|---|
| KIOM-2012b(1) | 0 | 10 | 50 | 100 |
| Saururi chinensis Baill. extract | 0 | 3.3 | 16.7 | 33.3 |
| Curcumae Longae Rhizoma extract | 0 | 3.3 | 16.7 | 33.3 |
| Polygalae Radix extract | 0 | 2 | 10 | 20 |
| Acori Gramineri Rhizoma extract | 0 | 1.4 | 6.6 | 13.4 |
| KIOM-2012b(2) | 0 | 10 | 50 | 100 |
| Saururi chinensis Baill. extract | 0 | 3.3 | 16.7 | 33.3 |
| Curcumae Longae Rhizoma extract | 0 | 2 | 10 | 20 |
| Polygalae Radix extract | 0 | 3.3 | 16.7 | 33.3 |
| Acori Gramineri Rhizoma extract | 0 | 1.4 | 6.6 | 13.4 |
| KIOM-2012b(3) | 0 | 10 | 50 | 100 |
| Saururi chinensis Baill. extract | 0 | 3.3 | 16.7 | 33.3 |
| Curcumae Longae Rhizoma extract | 0 | 1.4 | 6.6 | 13.4 |
| Polygalae Radix extract | 0 | 3.3 | 16.7 | 33.3 |
| Acori Gramineri | 0 | 2 | 10 | 20 |
| Rhizoma extract | | | | |

Then, the medium was removed from the plate and DMEM medium containing only 10 µg/mL insulin was added thereto, and cultured for two days. Subsequently, the resultant was cultured for 8 days while replacing with DMEM medium containing only FBS at two day intervals.

Upon completion of the culture, the resulting cells were collected, washed with PBS, and fixed with 10% formalin. The fixed cells were stained with Oil Red O and washed with 70% isopropyl alcohol. The washed cells were dried, added with 100% isopropyl alcohol to dissolve the Oil Red O stained in the cells. The absorbance of the resulting cells was measured at 490 nm, and their content of fat accumulated in the body was calculated based on the measured absorbances. In particular, the fat content was indicated as a percentage relative to the average absorbance value of the control group.

Meanwhile, the cell viability of the differentiated cells was measured via water-soluble tetrazolium (WST) method, the sodium salt of 4-[3-(4iodophenyl)-2-(4-nitrophenyl)-2H-5-tetrazolio]-1,3-benzene disulfonate, and evaluated the cell toxicities of each of the extract of the mixture and the individual extracts. Roughly, the culture-completed cells were treated with WST solution (10 µL/100 µL), cultured further for 1 hour, and the amount of formazan released as a culture liquid was calculated based on the absorbance at 520 nm, thereby measuring the level of the live cells, and the result was indicated as a ratio relative to the value of the control group.

First, upon analysis of the inhibitory effect of the hot-water extract on fat accumulation, as illustrated in FIGS. 1A through 1C, it was confirmed that each of the extract of the mixture (KIOM-2012Ob(1), KIOM-2012Ob(2), and KIOM-2012Ob(3)) reduced fat content in a concentration-dependent manner at concentrations of 50 µg/mL and 100 µg/mL, whereas, among the individual extracts, only the Polygalae Radix extract reduced the fat content similar to that of the extract of the mixture, but Saururi chinensis Baill. extract, Curcumae Longae Rhizoma extract, and Acori Gramineri Rhizoma extract failed to show any noticeable reduction in fat content regardless of their treated concentrations. Additionally, the fat reduction rates when treated with each of the extract of the mixture (KIOM-2012Ob(1), KIOM-2012Ob(2), and KIOM-2012Ob(3)) at a concentration of 50 µg/mL were 10, 13 and 17%, respectively, and those treated at a concentration of 100 µg/mL were 25%, 28%, and 26%, respectively.

Additionally, upon analysis of the cell toxicity of the hot-water extract, as illustrated in FIGS. 1D through 1F, it was confirmed that each of the extract of the mixture (KIOM-2012Ob(1), KIOM-2012Ob(2), and KIOM-2012Ob(3)) reduced the number of cells in a concentration-dependent manner at concentrations of 50 µg/mL and 100 µg/mL, whereas, among the individual extracts, only the Polygalae Radix extract singificantly reduced the number of cells, but Saururi chinensis Baill. extract, Curcumae Longae Rhizoma extract, and Acori Gramineri Rhizoma extract failed to show a reduction in the number of cells regardless of their treated concentrations.

In addition, upon analysis of the inhibitory effect of the warm-water extract on the fat accumulation, as illustrated in FIGS. 2A through 2C, it was confirmed that each of the extract of the mixture (KIOM-2012Ob(1), KIOM-2012Ob(2), and KIOM-2012Ob(3)) reduced fat content in a concentration-dependent manner at concentrations of 50 µg/mL and 100 µg/mL, whereas, among the individual extracts, only the Polygalae Radix extract reduced the fat content similar to that of he extract of the mixture, but Saururi chinensis Baill. extract, Curcumae Longae Rhizoma extract, and Acori Gramineri Rhizoma extract failed to show any noticeable reduction in fat content regardless of their treated concentration.

Additionally, upon analysis of the cell toxicity of the hot-water extract, as illustrated in FIGS. 2D through 2F, it was confirmed that each of the extract of the mixture (KIOM-2012Ob(1), KIOM-2012Ob(2), and KIOM-2012Ob(3)) reduced the number of cells in a concentration-dependent manner at concentrations of 50 µg/mL and 100 µg/mL, whereas, among the individual extracts, only the Polygalae Radix extract singificantly reduced the number of cells, but Saururi chinensis Baill. extract, Curcumae Longae Rhizoma extract, and Acori Gramineri Rhizoma extract failed to show a reduction in the number of cells regardless of their treated concentrations.

Finally, upon analysis of the inhibitory effect of the ethanol extract on the fat accumulation, as illustrated in FIGS. 3A through 3C, it was confirmed that each of the extract of the mixture (KIOM-2012Ob(1), KIOM-2012Ob(2), and KIOM-2012Ob(3)) reduced fat content in a concentration-dependent manner at concentrations of 10 µg/mL, 50 µg/mL, and 100 µg/mL, whereas, among the individual extracts, only the Polygalae Radix extract reduced the fat content similar to that of the extract of the mixture, but Saururi chinensis Baill. extract, Curcumae Longae Rhizoma extract, and Acori Gramineri Rhizoma extract failed to show any noticeable reduction in fat content regardless of their treated concentration.

Additionally, upon analysis of the cell toxicity of the ethanol extract, as illustrated in FIGS. 3D through 3F, it was confirmed that each of the extract of the mixture (KIOM-2012Ob(1), KIOM-2012Ob(2), and KIOM-2012Ob(3)) reduced the number of cells in a concentration-dependent manner at a concentration of 100 µg/mL, whereas, among the individual extracts, only the Polygalae Radix extract singificantly reduced the number of cells at concentrations of 50 µg/mL and 100 µg/mL, but Saururi chinensis Baill. extract, Curcumae Longae Rhizoma extract, and Acori Gramineri Rhizoma extract failed to show a reduction in the number of cells regardless of their treated concentrations.

Consequently, based on the above results of the extract of the mixture (the hot-water extract, the warm-water extract, and the ethanol extract), it was confirmed that the ethanol extract has the most superior effects in terms of the inhibition of fat accumulation and safety of cells.

### Example 3: In Vivo Anti-obesity Effects of Individual Extracts of Saururi chinensis Baill., Curcumae Longae Rhizoma, Polygalae Radix, and Acori Gramineri Rhizoma and Extract of the mixture

From the result of Example 2, it was confirmed that ethanol extracts of Saururi chinensis Baill., Curcumae Longae Rhizoma, Polygalae Radix, and Acori Gramineri Rhizoma have *excellent in vitro* effects in terms of the inhibition of fat accumulation and safety of cells. Accordingly, the present example was performed in order to confirm whether the ethanol extracts are still effective under *in vivo* condition. To this end, mice were bred while feeding them with the individual extracts and the extract of the mixture obtainded via ethanol extraction. The body weight, the amount of dietary intake, and fat weight of the thus-bred mice were measured, and their liver tissues and kidney tissues were analyzed by comparison.

### Example 3-1: Obtaining Mice Bred under Separate Breeding Conditions

One hundred seventy 5-week old male C57BL/6N mice were purchased, adapted for a week, and bred while feeding them with 60% high fat diet (HFD) for 5 weeks. The thus-bred mice were divided into 17 groups (10 mice/group) and bred for additional 6 weeks while feeding them with different feeds containing different test substances (Table 3). In particular, the positive control group was determined to be treated with Xenical™, an agent for treating obesity, and the extract of the mixture were administered into the normal diet group (1X) and excess diet group (4X). Additionally, the breeding room was set at a temperature of 22 ± 1°C with a humidity of 50 ± 5%, and the light-dark cycle was controlled at 12 hour intervals. The 10% high fat diet and 60% high fat diet were purchase from the Central Lab. (Animal Inc., Korea).

**[Table 3] Diet Conditions for Mice**

| **Test Group** | **Name** | **High Fat Diet Rate** | **Test Substance** | |
|---|---|---|---|---|
| | | | **Kind** | **Dose** (mg/kg) |
| 1 | normal control | 10% | - | - |
| 2 | negative control | 60% | - | - |
| 3 | positive control | 60% | Xenical™ | 62.5 |
| 4 | Saururi chinensis Baill. (1X) | 60% | Saururi chinensis Baill. extract | 36.4 |
| 5 | Saururi chinensis Baill. (4X) | 60% | Saururi chinensis Baill. extract | 145.6 |
| 6 | Curcumae Longae Rhizoma (1X) | 60% | Curcumae Longae Rhizoma extract | 278.5 |
| 7 | Curcumae Longae Rhizoma (4X) | 60% | Curcumae Longae Rhizoma extract | 111.4 |
| 8 | Polygalae Radix (1X) | 60% | Polygalae Radix extract | 97.2 |
| 9 | Polygalae Radix (4X) | 60% | Polygalae Radix extract | 388.8 |
| 10 | Acori Gramineri Rhizoma (1X) | 60% | Acori Gramineri Rhizoma extract | 50.9 |
| 11 | Acori Gramineri Rhizoma (4X) | 60% | Acori Gramineri Rhizoma extract | 203.6 |
| 12 | KIOM-2012Ob(1) (1X) | 60% | KIOM-2012Ob(1) | 106.2 |
| 13 | KIOM-2012Ob(1) (4X) | 60% | KIOM-2012Ob(1) | 424.8 |
| 14 | KIOM-2012Ob(2) (1X) | 60% | KIOM-2012Ob(2) | 106.2 |
| 15 | KIOM-2012Ob(2) (4X) | 60% | KIOM-2012Ob(2) | 424.8 |
| 16 | KIOM-2012Ob(3) (1X) | 60% | KIOM-2012Ob(3) | 146.4 |
| 17 | KIOM-2012Ob(3) (4X) | 60% | KIOM-2012Ob(3) | 584.4 |

### Example 3-2: Evaluation of Body Weight

The body weight of the 17 different kinds of experimental mice bred in Example 3-1 was measured and analyzed by comparison (FIG. 4). As illustrated in FIG. 4, experimental group 9 (Polygalae Radix (4X)) showed the highest decrease in body weight closest to that of the normal control group, followed by experimental group 15 (KIOM-2012Ob(2) (4X)) and experimental group 17 (KIOM-2012Ob(3) (4X)). The remaining experimental groups also showed a lower level compared to that of the negative control group, thus generally showing the effect of decrease of body weight.

However, in the case of experimental group 9 (Polygalae Radix (4X)), four out of 10 mice in the group died during the 6 week period, and one of them showed a significant deterioration in its health condition thus showing the damage caused by toxicity. In contrast, experimental group 15 (KIOM-2012Ob(2) (4X)) and experimental group 17 (KIOM-2012Ob(3) (4X)) did not show any damage caused by toxicity.

### Example 3-3: Evaluation of the Amount of Dietary Intake

The amount of dietary intake of the 17 different kinds of experimental mice bred in Example 3-1 was measured and analyzed by comparison (FIG. 5). As illustrated in FIG. 5, no significance was observed except experimental group 9 (Polygalae Radix (4X)), although there was a difference in the amount of dietary intake among the experimental groups.

### Example 3-4: Evaluation of the Amount of Fat Weight

The 17 different kinds of experimental mice bred in Example 3-1 were fasted for 5 hours to allow them to digest all the foods intaken, sacrificed by cervial dislocation, and the abdominal fat and subcutaenous fat were collected from each mouse. The thus-obtained abdominal fat and subcutaenous fat were washed with saline solution, placed on a filter paper to remove water therefrom, and the total weight of the fats was measured and analyzed by comparison (FIG. 6). As illustrated in FIG. 6, experimental group 9 (Polygalae Radix (4X)) and experimental group 15 (KIOM-2012Ob(2) (4X)) showed a significant decrease in their fat weight compared to that of the negative control group. Additionally, in experimental groups 12 (KIOM-2012Ob(1) (1X)), 14 (KIOM-2012Ob(2) (1X)) and 16 (KIOM-2012Ob(3) (1X)), which were treated with the same level of the extract of the mixture, the fat weight increased in the order of experimental groups 14, 16 and 12, whereas in experimental groups 13 (KIOM-2012Ob(1) (4X)), 15(KIOM-2012Ob(2) (4X)) and 17(KIOM-2012Ob(3) (4X)), the fat weight increased in the order of experimental groups 15, 17, and 13. Accordingly, considering the contents of Saururi chinensis Baill., Curcumae Longae Rhizoma, Polygalae Radix and Acori Gramineri Rhizoma included in each of the extract of the mixture, it was analyzed that the Polygalae Radix has the most significant inhibitory effect against fat increase, followed by Curcumae Longae Rhizoma, whereas Acori Gramineri Rhizoma has the least inhibitory effect against fat increase.

### Example 3-5: Pathological Analysis of Liver Tissues

Liver tissues were collected from the mice sacrificed in Example 3-4, fixed in formalin, and then subjected to pathological analysis thereby analyzing the levels of microvescicular steatosis and chronic inflammation included in the liver tissues by comparison (FIG. 7). As illustrated in FIG. 7A, the microvescicular steatosis included in the liver tissues showed low values both in experimental groups 8 and 9, which were treated with the Polygalae Radix extract, and experimental groups 12 through 17, which were treated with the extract of the mixture, compared to that of the negative control group, thus confirming the significant decrease of the level of the microvescicular steatosis included in the liver tissues.

Additionally, as illustrated in FIG. 7B, the level of the chronic inflammation in the liver tissues was higher in all experimental groups than the negative control group, and in particular, the highest in experimental groups 8 and 9, which were treated with the Polygalae Radix extract. In contrast, the mice in experimental groups 12 through 17, which were treated with the extract of the mixture, showed a lower level of the chronic inflammation than the mice treated with the Polygalae Radix extract.

### Example 3-6: Pathological Analysis of Kidney Tissues

Kidney tissues were collected from the mice sacrificed in Example 3-4, fixed in formalin, and then subjected to pathological analysis thereby analyzing the levels of microvescicular steatosis and chronic inflammation included in the kidney tissues by comparison (FIG. 8). As illustrated in FIG. 8, the level of the chronic inflammation in the kidney tissues was higher in all experimental groups than the negative control group and even higher than the normal control group, and in particular, relatively higher in experimental group 9, which was treated with the Polygalae Radix extract. In contrast, the mice in experimental groups 12 through 17, which were treated with the extract of the mixture, showed an equal or lower level of the chronic inflammation than the mice treated with the Polygalae Radix extract.

Consequently, based on the results from Examples 3-2 through 3-6, the Polygalae Radix extract and the extract of the mixture showed *in vivo* inhibitory effects against obesity. Unlike the Polygalae Radix extract, which showed excellent inhibitory effect agasint obesity but has serious toxicities, the extract of the mixture showed improved safety along with the relatively superior inhibitory effects against obesity.

### Example 4: Effects of the Extract of the mixture derived from Saururi chinensis Baill., Curcumae Longae Rhizoma and Polygalae Radix

From the result of Example 3-4, it was confirmed that Acori Gramineri Rhizoma, among the components included in the extract of the mixture, has the least inhibitory effect agasint fat increase. Accordingly, the present example was performed in order to confirm whether the extract of the mixture obtained from a sample including only Saururi chinensis Baill., Curcumae Longae Rhizoma, and Polygalae Radix, exclusive of Acori Gramineri Rhizoma, has an inhibitory effect against obesity.

### Example 4-1: Obtaining Natural Extracts

Mixtures essentially including ground Saururi chinensis Baill., Curcumae Longae Rhizoma, and Polygalae Radix, and selectively including Acori Gramineri Rhizoma and clover were obtained (Table 4). The mixtures were subjected to extractions for 24 hours after adding 300 mL of 25% ethanol to each of the mixtures while maintaining the temperature at 38°C, and the respective extract of the mixture was obtained therefrom. Each of the thus-obtained extracts was subjected to filtration to obtain a liquid component, and the liquid component was lyophilized to obain powdered extracts, which were then dissolved in distilled water at a concentration of 100 mg/mL to be used later as samples for experiments.

Additionally, a green tea extract to be used in a comparative group was obtained in the same manner.

**[Table 4] Composition Ratio of Extract of the mixture**

| **Extract** | **Saururi chinensis Baill.** | **Curcumae Longae Rhizoma** | **Polygalae Radix** | **Acori Gramineri Rhizoma** | **Clover** |
|---|---|---|---|---|---|
| K | 8g | 8g | 8g | - | - |
| KS | 8g | 8g | 8g | 4g | - |
| KC | 8g | 8g | 8g | - | 4g |

### Example 4-2: Obtaining Mice Bred Under Various Conditions

One hundred twenty six 5-week old male C57BL/6N mice were purchased, adapted for a week, and bred while feeding them with 60% high fat diet (HFD) for 8 weeks. The thus-bred mice were divided into 14 groups (9 mice/group) and bred for additional 6 weeks while feeding them with different feeds containing different test substances (Table 5). In particular, the breeding room was set at a normal temperature of 22 ± 1°C with a humidity of 50 ± 5%, and the light-dark cycle was controlled at 12 hour intervals. Saline solution was used as an excipient.

**[Table 5] Diet Conditions for Mice**

| **Test Group** | **Name** | **High Fat Diet Rate** | **Test Substance** | |
|---|---|---|---|---|
| | | | **Kind** | **Dose** (mg/kg) |
| 21 | normal control | 10% | - | - |
| 22 | negative control | 60% | - | - |
| 23 | positive control 1, PC 1 | 60% | green tea extract | 200 |
| 24 | positive control 1, PC2 | 60% | L-camitine | 30 |
| 25 | positive control 1, PC3 | 60% | Xenical™ | 25 |
| 26 | positive control 1, PC4 | 60% | Xenical™ | 75 |
| 27 | K150 | 60% | K | 150 |
| 28 | K300 | 60% | K | 300 |
| 29 | KS 150 | 60% | KS | 150 |
| 30 | KS 300 | 60% | KS | 300 |
| 31 | KC 150 | 60% | KC | 150 |
| 32 | KC 300 | 60% | KC | 300 |
| 33 | KCL | 60% | KC + L-camitine | 300 + 30 |
| 34 | KSL | 60% | KS + L-camitine | 300 + 30 |

### Example 4-3: Evaluation of Body Weight

The body weight of the experimental mice of 14 different kinds bred in Example 4-2 was measured and analyzed by comparision (FIG. 9). As illustrated in FIG. 9, the body weight of all the experimental groups (experimental groups 27 through 34) was lower than that of the negative control group, and experimental group 34 (KSL) showed the highest reduction in body weight to a level closest to that of the normal control group, followed by experimental groups 28 (K 300), 30 (KS 300), 33 (KCL), 29 (KS 150), 27 (K 150), 31 (KC 150), and 32 (KC 300), in that order.

From the above results, it was confirmed that the extract of the mixture obtained from a sample including Saururi chinensis Baill., Curcumae Longae Rhizoma, and Polygalae Radix can exhibit the inhibitory effect against increase of body weight.

### Example 4-4: Evaluation of Dietary Intake

The amount of dietary intake of the experimental mice of 14 different kinds bred in Example 4-2 was measured and analyzed by comparision (FIG. 10). As illustrated in FIG. 10, no significance was observed although there was a difference in the amount of dietary intake among the experimental groups.

### Example 4-5: Evaluation of Fat Weight

The 14 different kinds of the experimental mice bred in Example 4 were fasted for 5 hours to allow them to digest all the foods intaken, sacrificed by cervial dislocation, and the abdominal gonadal fat and mesenteric fat were collected from each mouse. The thus-obtained abdominal gonadal fat and mesenteric fat were washed with saline solution, placed on a filter paper to remove water therefrom, and each and the total weight of the fats was measured and analyzed by comparison (FIGS. 11A through 11D).

As illustrated in FIG. 11A, the level of the abdominal gonadal fat in all the experimental groups (experimental groups 27 through 34) was lower than that of the negative control group, and experimental group 28 (K 300) showed the highest reduction in fat weight to a level closest to that of the normal control group, followed by experimental groups 34 (KSL), 30 (KS 300), 33 (KCL), 27 (K 150), 32 (KC 300), 29 (KS 150) and 31 (KC 150), in that order.

As illustrated in FIG. 11B, the level of the mesenteric fat in all experimental groups (experimental groups 27 through 34) was lower than that of the negative control group, and experimental group 30 (KS 300) showed the highest reduction in fat weight to a level closest to that of the normal control group, followed by experimental groups 34 (KSL), 28 (K 300), 33 (KCL), 32 (KC 300), 29 (KS 150), 27 (K 150) and 31 (KC 150), in that order.

As illustrated in FIG. 11C, the level of the subcutaneous fat in all the experimental groups (experimental groups 27 through 34) was lower than that of the negative control group, and experimental group 28 (K 300) showed the highest reduction in fat weight to a level closest to that of the normal control group, followed by experimental groups 34 (KSL), 30 (KS 300), 33 (KCL), 29 (KS 150), 27 (K 150), 32 (KC 300) and 31 (KC 150), in that order.

As illustrated in FIG. 11D, the level of the total fat in all the experimental groups (experimental groups 27 through 34) was lower than that of the negative control group, and experimental group 28 (K 300) showed the highest reduction in fat weight to a level closest to that of the normal control group, followed by experimental groups 34 (KSL), 30 (KS 300), 33 (KCL), 27 (K 150), 29 (KS 150), 32 (KC 300), and 31 (KC 150), in that order.

Consequently, based on the above results (FIGS. 11A through 11D), it was confirmed that the extract of the mixture obtained from a sample including Saururi chinensis Baill., Curcumae Longae Rhizoma, and Polygalae Radix can exhibit the inhibitory effect against fat accumulation.

### Example 4-6: Evaluation of Ghrelin Level

The level of ghrelin, known as a hunger hormone secreted in the stomach, was evaluated in details in terms of activated ghrelin, desacyl-ghrelin, and ghrelin ratio, using the experimental mice of 14 different kinds bred in Example 4-2.

First, the level of the activated ghrelin present in the blood plasma was measured using a ghrelin measurement kit (Active Ghrelin ELISA kit, SCETI, Japan). Roughly, the blood collected from the mice of each experimental group was added into a tube containing EDTA and aptotinin and mixed. The mixture was centrifuged to obtain blood plasma. The thus-obtained plasma was added with 1 mol/L HCl in a volume corresponding to 10% of the plasma volume to thereby prepare a sample. The thus-prepared sample was added to the ghrelin measurement kit and reacted. The absorbance of the resultant was measured at 450 nm, and the level of the activated ghrelin was calculated based on the measured absorbance value (FIG. 12A).

As illustrated in FIG. 12A, regarding the level of activated ghrelin, experimental group 32 (KC 300) showed a higher level than the negative control group, and experimental groups 27 (K 150) and 34 (KSL) showed similar levels to that of the negative control group. Experimental groups 29 (KS 150), 28 (K 300), 33 (KCL), 30 (KS 300) and 31 (KC 150) showed a lower level than the negative control group, and experimental group 29 (KS 150) showed the lowest level.

Then, the level of desacyl-ghrelin present in blood plasma was measured in the same manner as described above except that desacyl-ghrelin measurement kit (desacyl-Ghrelin ELISA kit, SCETI, Japan) was used instead of the ghrelin measurement kit (Active Ghrelin ELISA kit, SCETI, Japan) (FIG. 12B).

As illustrated in FIG. 12B, regarding the level of desacyl-ghrelin, experimental group 32 (KC 300) showed a lower level than the negative control group, experimental groups 29 (KS 150) and 34 (KSL) showed a similar level to that of the negative control group, and experimental groups 27 (K 150), 28 (K 300), 33 (KCL), 30 (KS 300), and 31 (KC 150) showed higher levels than the negative control group, wherein experimental group 33 (KCL) showed the highest level.

Finally, the ghrelin ratio was calculated as a percentage of the activated ghrelin relative to the level ofthe measured desacyl-ghrelin (FIG. 12C).

As illustrated in FIG. 12C, experimental group 32 (KC 300) showed a higher level of the ghrelin ratio than the negative control group, experimental group 27 (K 150) showed a similar level to that of the negative control group, and experimental groups 34 (KSL), 29 (KS 150), 28 (K 300), 33 (KCL), 30 (KS 300) and 31 (KC 150) showed lower levels of desacyl-ghrelin than the negative control group, wherein experimental group 33 (KCL) showed the lowest level.

Consequently, based on the above results (FIGS. 12A through 12C), it was confirmed that the extract obtained from the mixture comprising Saururi chinensis Baill., Curcumae Longae Rhizoma, and Polygalae Radix showed similar or lower levels of activated ghrelin, desacyl-ghrelin, and ghrelin ratio than the negative control group, thus suggesting that the anti-obesity effect of the extract of the mixture was apparently not due to the inhibition of appetite.

### Example 4-7: Evaluation of Leptin

The level of leptin in the blood plasma was evaluated using the experimental mice of 14 different kinds bred in Example 4-2.

Specifically, the level of leptin present in the blood plasma was measured using a leptin measurement kit (Mouse and Rat Leptin ELISA kit, mediagnost, Germany). Roughly, the blood collected from the mice of each experimental group was added into a tube containing EDTA and aptotinin, mixed, and centrifuged to thereby obtain blood plasma. The thus-obtained blood plasma was added with a dilution buffer included in the leptin measurement kit, and the diluted solution was added to the leptin measurement kit and reacted. The absorbance of the resultant was measured at 450 nm, and the level of leptin was calculated based on the measured absorbance value (FIG. 13).

As illustrated in FIG. 13, regarding the level of leptin in the blood plasma, all the experimental groups (experimental groups 27 through 34) showed lower levels than the negative control group, and experimental group 28 (K 300) showed the highest reduction in leptin level to a level closest to that of the normal control group, followed by experimental groups 33 (KCL), 34 (KSL), 30 (KS 300), 29 (KS 150), 27 (K 150), 32 (KC 300), and 31 (KC 150), in that order.

In fact, it is generally known that obesity-induced experimental animals show an increased leptin resistance thereby elevating the leptin level in the blood. Experimental group 28 (K 300), which were treated with the extract obtained from the mixture consisting of Saururi chinensis Baill., Curcumae Longae Rhizoma, and Polygalae Radix, showed the lowest level of leptin, thus implying the lowest leptin resistance. Accordingly, it was confirmed that the extract of the mixture is effective in preventing obesity from occurring.

### Example 4-8: Evaluation of Level of Blood Components

The levels of blood components including triglycerides, total cholesterol, LDL-cholesterol, HDL-cholesterol, glucose, creatinine, urea, LDH, ALP, GPT, GOT, etc., were evaluated according to the known method using the experimental mice of 14 different kinds bred in Example 4-2 (FIGS. 14A through 14K).

As illustrated in FIG. 14A, regarding the level of triglycerides in the blood, only experimental group 34 (KSL) showed a lower level than the negative control group, and the remaining experimental groups showed similar or higher levels than the negative control group, wherein experimental group 32 (KC 300) showed the highest level.

As illustrated in FIG. 14B, regarding the level of total cholesterol in the blood, experimental groups 27 (K 150) and 34 (KSL) showed lower levels than the negative control group, experimental groups 28 (K 300) and 30 (KS 300) showed similar levels to that of the negative control group, experimental groups 29 (KS 150), 33 (KCL), 32 (KC 300) and 31 (KC 150) showed higher levels than the negative control group, and experimental group 33 (KCL) showed the highest level.

As illustrated in FIG. 14C, regarding the level of LDL-cholesterol in the blood, experimental groups 31 (KC 150), 32 (KC 300) and 33 (KCL) showed higher levels than the negative control group, experimental groups 29 (KS 150) and 30 (KS 300) showed similar levels to the negative control group, experimental groups 27 (K 150), 28 (K 300) and 34 (KSL), and experimental group 34 (KSL) showed the lowest level.

As illustrated in FIG. 14D, regarding the level of HDL-cholesterol in the blood, only experimental group 28 (K 300) showed a lower level than the negative control group, and the remaining experimental groups showed similar or higher levels than the negative control group, and experimental group 31 (KC 150) showed the highest level.

As illustrated in FIG. 14E, regarding the level of glucose in the blood, experimental groups 28 (K 300) and 29 (KS 150) showed lower levels than the negative control group, experimental group 34 (KSL) showed a similar level to that of the negative control group, experimental groups 27 (K 150), 31 (KC 150), 32 (KC 300), 33 (KCL) and 30 (KS 300) showed higher levels than the negative control group, and experimental group 32 (KC 300) showed the highest level.

As illustrated in FIG. 14F, regarding the level of creatinine in the blood, all the experimental groups (experimental groups 27 through 34) showed similar or higher levels than the negative control group. Specifically, experimental groups 29 (KS 150) and 31 (KC 150) showed higher levels than the negative control group, wherein experimental group 34 (KSL) showed the highest level.

As illustrated in FIG. 14G, regarding the level of urea in the blood, all the experimental groups (experimental groups 27 through 34) showed higher levels than the negative control group, and specifically, experimental group 33 (KCL) showed the highest improvement in urea level, followed by experimental groups 30 (KS 300), 34 (KSL), 27 (K 150), 31 (KC 150), 28 (K 300), 29 (KS 150) and 32 (KC 300), in that order.

As illustrated in FIG. 14H, regarding the level of lactate dehydrogenase (LDH) in the blood, all the experimental groups (experimental groups 27 through 34) showed similar or higher levels than the negative control group. Specifically, experimental groups 30 (KS 300) and 34 (KSL) showed similar levels to that of the negative control group, and the remaining experimental groups showed higher levels than the negative control group, wherein experimental group 32 (KC 300) showed the highest level.

As illustrated in FIG. 14I, regarding the level of alkaline phosphatase (ALP) in the blood, only experimental group 29 (KS 150) showed a higher level than the negative control group, and the remaining experimental groups showed similar or higher levels than the negative control group, wherein experimental group 31 (KC 150) showed the lowest level.

As illustrated in FIG. 14J, regarding the level of glutamic-pyruvic transaminase (GPT) in the blood, all the experimental groups (experimental groups 27 through 34) showed similar or higher levels than the negative control group. Specifically, experimental groups 27 (K 150), 28 (K 300) and 30 (KS 300) showed higher levels than the negative control group, wherein experimental group 31 (KC 150) the highest level.

As illustrated in FIG. 14K, regarding the level of glutamic-oxaloacetic transaminase (GOT) in the blood, all the experimental groups (experimental groups 27 through 34) showed similar or higher levels than the negative control group. Specifically, experimental groups 29 (KS 150) and 34 (KSL) showed similar levels to that of the negative control group, and the remaining experimental groups showed higher levels than the negative control group, wherein experimental group 31 (KC 150) showed the highest level.

As illustrated in FIGS. 14I through 14K, the experimental groups treated with the extract obtained from the mixture consisting of Saururi chinensis Baill., Curcumae Longae Rhizoma, and Polygalae Radix provided in the present invention, in general failed to show significance regarding the ALP, GPT and GOT levels in the blood compared to the control group thus implying that the extract of the mixture does not exhibit hepatotoxicity.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A pharmaceutical composition for the prevention or treatment of obesity comprising an extract of a mixture comprising Saururi chinensis Baill., Curcumae Longae Rhizoma, and Polygalae Radix; or a fraction thereof

2. The pharmaceutical composition of claim 1, wherein the extract is an extract of a mixture comprising Saururi chinensis Baill., Curcumae Longae Rhizoma, and Polygalae Radix mixed at a ratio of 1 : 0.2 through 1 : 0.2 through 1 (w/w/w) therein.

3. A pharmaceutical composition for the prevention or treatment of obesity comprising an extract of a mixture comprising Saururi chinensis Baill., Curcumae Longae Rhizoma, Polygalae Radix, and Acori Gramineri Rhizoma; or a fraction thereof

4. The pharmaceutical composition of claim 3, wherein the extract is an extract of a mixture comprising Saururi chinensis Baill., Curcumae Longae Rhizoma, Polygalae Radix, and Acori Gramineri Rhizoma mixed at a ratio of 1 : 0.2 through 1 : 0.2 through 1 : 0.2 through 1 (w/w/w/w) therein.

5. The pharmaceutical composition of claim 1 or claim 3, wherein the extract is obtained by extracting the mixture with 25% ethanol.

6. A food composition for the prevention or improvement of obesity comprising an extract of a mixture comprising a Saururi chinensis Baill., a Curcumae Longae Rhizoma, and a Polygalae Radix; or a fraction thereof

7. The food composition of claim 6, wherein the extract is an extract of a mixture comprising Saururi chinensis Baill., Curcumae Longae Rhizoma, and Polygalae Radix mixed at a ratio of 1 : 0.2 through 1 : 0.2 through 1 (w/w/w) therein.

8. A food composition for the prevention or improvement of obesity comprising an extract of a mixture comprising Saururi chinensis Baill., Curcumae Longae Rhizoma, Polygalae Radix, and Acori Gramineri Rhizoma; or a fraction thereof

9. The food composition of claim 8, wherein the extract is an extract of a mixture comprising Saururi chinensis Baill., Curcumae Longae Rhizoma, Polygalae Radix, and Acori Gramineri Rhizoma mixed at a ratio of 1 : 0.2 through 1 : 0.2 through 1 : 0.2 through 1 (w/w/w/w) therein.

10. The food composition of claim 6 or claim 8, wherein the extract is obtained by extracting the mixture with 25% ethanol.

11. The food composition of claim 6 or claim 8, wherein the fraction is obtained by applying the extract to a method selected from the group consisting of solvent fractionation, ultrafiltration fractionation, chromatography fractionation, and a combination thereof

12. A method of manufacturing an extract from a mixture comprising Saururi chinensis Baill., Curcumae Longae Rhizoma, and Polygalae Radix, comprising extracting the mixture with water, a C₁ ∼ C₄ alcohol, or a mixed solvent thereof

13. The method of claim 12, wherein the mixture comprises Saururi chinensis Baill., Curcumae Longae Rhizoma, and Polygalae Radix at a ratio of 1 : 0.2 through 1 : 0.2 through 1 (w/w/w).

14. A method of manufacturing an extract from a mixture comprising Saururi chinensis Baill., Curcumae Longae Rhizoma, Polygalae Radix, and Acori Gramineri Rhizoma, comprising extracting the mixture with water, a C₁ ∼ C₄ alcohol or a mixed solvent thereof

15. The method of claim 14, wherein the mixture comprises Saururi chinensis Baill., Curcumae Longae Rhizoma, Polygalae Radix, and Acori Gramineri Rhizoma at a ratio of 1 : 0.2 through 1 : 0.2 through 1 : 0.2 through 1 (w/w/w/w).
